# EUROPEAN PATENT SPECIFICATION

(11) **EP 3 829 715 B1**
(45) Date of publication and mention of the grant of the patent: **03.12.2025**
(21) Application number: 19752877.1
(22) Date of filing: 01.08.2019
(51) Int. Cl.: A61P 15/08, A61K 31/7064, C07H 19/048

(54) **QUATERNARY AMMONIUM SALTS OF NICOTINIC ACID AND NICOTINAMIDE MONONUCLOETIDES AND RIBOSIDES AS ANTI-AGING AGENTS**
QUATERNÄRE AMMONIUMSALZE VON NIKOTINSÄURE UND NIKOTINAMIDMONONUKLEOTIDEN UND RIBOSIDEN ALS ANTI-AGING-MITTEL
SELS D'AMMONIUM QUATERNAIRES D'ACIDE NICOTINIQUE ET DE MONONUCLÉOTIDES DE NICOTINAMIDE ET DE RIBOSIDES EN TANT QU'AGENTS ANTI-ÂGE

(30) Priority: 01.08.2018 US 201862713046 P
(43) Date of publication of application: 09.06.2021
(73) Proprietor: Jumpstart Fertility Pty Ltd, Coogee NSW 2034 (AU); Life Biosciences, Inc., Boston MA 02116 (US)
(72) Inventor: MARCUCCIO, Sebastian Mario, Coogee, New South Wales 2034 (AU); JOYCE, Rohan David, Coogee, New South Wales 2034 (AU); WATHIER, Michel, Allston, Massachusetts 02133 (US); DOLLE, Roland, Boston, Massachusetts 02116 (US); TUCKER, Simon, Coogee, New South Wales 2034 (AU)
(74) Representative: Grund, Martin
(86) International application number: PCT/US2019/044697
(87) International publication number: WO 2020/028682

(56) References cited:
- WO-A1-2013/002880

## Description

### Field of the Disclosure

The present invention relates to quaternary salts of nicotinic acid mononucleotides and nicotinamide mononucleotides and compositions thereof useful in the treatment of disorder and diseases associated with aging.

### Background of the Disclosure

Aging is the result of complex interactions involving biological, physical, and biochemical processes that cause dysfunctions in cells and organs which manifests in a variety of diseases and other outcomes. For example, female fecundity is markedly sensitive to the effects of ageing. For example, the USA Centers for Disease Control has reported that the percentage of assisted reproductive technology (ART) associated pregnancies and births percentages declined steadily among women in their mid-30s onward from approximately 25% of ART cycles resulting in singleton live births to 14% by the age of 40 (Centers for Disease Control and Prevention, American Society for Reproductive Medicine, Society for Assisted Reproductive Technology. 2011 Assisted Reproductive Technology National Summary Report. Atlanta (GA): US Dept of Health and Human Services; 2013). This trend is markedly increased above the age of 40 with the CDC reporting that women older than age 44 have a very low likelihood of success. The percentages of live births and singleton live births declined to about 1% in this group. It is generally considered that a woman's age is the most important factor affecting the chance of a live birth when her own eggs (oocytes) are used.

It is understood that the qualitative deterioration of oocytes due to aging is a fundamental factor in the decline in fertility. In older women, for example, the oocytes are reported to be susceptible to abnormal chromosome division, exhibit decreased mitochondrial quality, low ATP production, increased oxidative stress, and decreased antioxidant levels (Nelson SM, Telfer EE, Anderson RA. The ageing ovary and uterus: new biological insights. Hum Reprod Update. 2013;19:67-83.; Wilding M. Potential long-term risks associated with maternal aging (the role of the mitochondria). Fertil. Steril. 2015;103:1397-401; 3. Meldrum DR, Casper RF, Diez-Juan A, Simon C, Domar AD, Frydman R. Aging and the environment affect gamete and embryo potential: can we intervene? Fertil. Steril. 2016;105:548-59).

For all of the foregoing reasons, the oocyte represents an excellent target tissue for the evaluation of therapeutic modalities that are expected to have an impact upon the ageing process and, furthermore, offer the prospect of addressing age-related infertility.

One such possible therapeutic modality for treating ageing comprises agents which boost therapeutic levels of NAD⁺. NAD⁺ is an essential component of cellular processes necessary to support various metabolic functions. The classic role of NAD⁺ is a co-enzyme that catalyzes cellular redox reactions, becoming reduced to NADH, in many fundamental metabolic processes, such as glycolysis, fatty acid beta oxidation, or the tricarboxylic acid cycle. In addition to playing these roles, NAD⁺ has a critical role as the substrate of NAD⁺-consuming enzymes such as poly-ADP-ribose polymerases (PARPs), sirtuins, and CD38/157 ectoenzymes. These NAD⁺-consuming enzymes have been known to mediate many fundamental cellular processes.

There are five major precursors and intermediates to synthesize NAD⁺: tryptophan, nicotinamide, nicotinic acid (NA), nicotinamide riboside (NR), and nicotinamide mononucleotide (NMN). NAD⁺ can be synthesized de novo by the conversion of the amino acid tryptophan through multiple enzymatic steps to nicotinic acid mononucleotide (NaMN). NaMN is converted to nicotinic acid dinucleotide (NaAD⁺) by NMN/NaMN adenylyltransferases (NMNATs) and then amidated to NAD⁺ by NAD⁺ synthetase.

In mammals, a major pathway of NAD⁺ biosynthesis is the salvage pathway from nicotinamide. Nicotinamide is converted to NMN, a key NAD⁺ intermediate, by nicotinamide phosphoribosyltransferase (NAMPT), the rate-limiting enzyme in this pathway. NMNATs then convert NMN into NAD⁺. NAMPT plays a critical role in regulating cellular NAD⁺ levels. On the other hand, nicotinic acid is converted to NaMN by nicotinic acid phosphoribosyltransferase (NPT). NR needs to be converted to NMN by nicotinamide ribose kinases, NMRK1 and NMRK2 (also known as NRK1 and NRK2), which phosphorylate NR. Maintenance of adequate NAD⁺ biosynthesis is paramount for cell survival and function. Derailment from normal NAD⁺ homeostasis substantially affects not only the NAD⁺/NADH pool required for redox reactions but also activities of NAD⁺-dependent enzymes for crucial cellular functions.

It is now becoming a consensus that NAD⁺ levels decline at cellular, tissue/organ, and organismal levels during the course of aging. Activities of NAD⁺-consuming enzymes are affected by this NAD⁺ decline, contributing to a broad range of age-associated pathophysiologies

Nicotinamide adenine dinucleotide is an enzyme co-factor that is essential for the function of several enzymes related to reduction-oxidation reactions and energy metabolism. (Katrina L. Bogan & Charles Brenner, Nicotinic Acid, Nicotinamide and Nicotinamide Riboside: A Molecular Evaluation of NAD+ Precursor Vitamins in Nutritions, 28, Annual Review of Nutrition 115 (2008)). NAD⁺ functions as an electron carrier in energy metabolism of amino acids, fatty acids and carbohydrates (Bogan & Brenner, Annu. Rev. Nutr. 2008, 28, 115-130). NAD⁺ is critical for redox reactions and as a substrate for signaling by the PARPs (poly adenosidediphophosphate-ribose polymerases) and the sirtuins (SIRT1 to SIRT7), in the regulation of DNA repair, energy metabolism, cell survival and circadian rhythms which have all been shown to be critical in the ageing process (Bronkowski, M.S. & Sinclair, D., Nat. Rev. Mole. Cell. Bio., 17, 679-690, (2016)). Raising NAD⁺ concentrations delays aging in yeast, files and mice (Mouchiroud et al. Cell 154, 464-471, (2014)). It has recently also been demonstrated that NAD⁺ directly regulates protein-protein interactions, the modulation of which may protect against cancer and radiation exposure as well as having a direct impact on aging (Li et al., Science 355, 1312-1317, 2017). Thus increasing bodies of evidence support the idea that interventions using NAD⁺ intermediates, such as NMN and NR, can bolster the system by restoring the available NAD⁺ and mitigate physiological decline associated with aging.

Although NAD⁺ can be synthesized de novo from the amino acid tryptophan, this process does not occur in all tissues, requiring most cells to rely on the salvage pathway (described above) for regenerating NAD⁺ from other intracellular intermediates, which are primarily made available through dietary sources (Christopher R. Martens, et al., Nat. Commun.9, 1286, (2018) and Bogan, K. L. & Brenner, C., Annu. Rev. Nutr. 28, 115-130, (2008)). Other NAD precursors like nicotinic acid and nicotinamide can also be administered to boost NAD cellular bioavailability. However, clinically relevant levels of nicotinic acid are associated with undesirable flushing at therapeutic doses (MacKay, D., Hathcock, J. &Guarneri, E., Nutr. Rev. 70, 357-366 (2012)). and nicotinamide does not reliably activate (and may even inhibit) sirtuins despite raising concentrations of NAD (Bitterman, K. J., et al., J. Biol. Chem. 277, 45099-45107 (2002); Guan, X., et al., PLoS One. 9, e107729 (2014); and Trammell, S. A. et al. Nat. Commun. 7, 12948 (2016)). Therefore, administration of nicotinic acid or nicotinamide is unlikely to be widely adopted for maintaining health and function with aging.

In contrast to nicotinic acid and nicotinamide, administration of NAD⁺ metabolites such as nicotinamide mononucleotide (NMN) or nicotinamide riboside (NR), appears to increase levels of NAD⁺ and improves multiple physiological functions in animal models (Yoshino, J. et al., Cell Metab. 14, 528-536 (2011); Mills, K. F. et al., Cell Metab. 24, 795-806 (2016); and Frederick, D. W. et al., Cell Metab. 24, 269-282 (2016)). At least one of these metabolites has been reported to be well tolerated in humans leading to elevation of NAD levels and improved physiological functions albeit that further studies are required to confirm the findings of this exploratory study (Christopher R. Martens, et al., Nat. Commun. 9, 1286, (2018)). Furthermore, a recent study showed that single doses of NR stimulated blood cellular NAD⁺ metabolism in healthy humans in a dose-dependent manner (Trammell, S. A. et al., Nat. Commun. 7, 12948 (2016)), showing the limitation of this metabolite. WO 2013/002880 A1 discloses a solution comprising an oocyte and NR. However, many of the known NAD⁺ metabolites are unstable in a variety of physiological environments and thus do not lend themselves to viable pharmaceutical drugs for administration to patients in need of such metabolites for boosting the NAD⁺ levels in said patients.

Given the central role that NAD⁺ plays in critical cellular and physiological pathways, developing novel stable agents with improved properties that can elevate NAD⁺ levels in disease states and/or during the aging process is necessary to improve the human condition.

### Summary of the Disclosure

A first aspect of the application relates to salts of Formula (I): 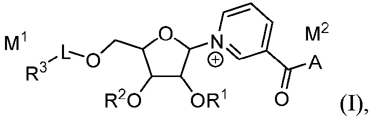
and enantiomers, stereoisomers, and tautomers thereof,
wherein
   A is NR^{a}R^{b} or O⁻;
   L is a bond or 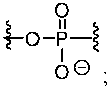 R¹ and R² are independently H, C₁-C₆alkyl, C₁-C₆haloalkyl, -C(O)C₁-C₆haloalkyl, (C₀-C₃alkylene)C(O)C₁-C₆alkyl, -C(O)OR^{a}, -C(O)NR^{a}R^{b},-[CH₂-CH₂-O]ₖ-R^{a}, - C(O)[CH₂-CH₂-O]ₖ-R^{a}, -[CH₂-CH₂-CH₂-O]ₖ-R^{a}, or -C(O)[CH₂-CH₂-CH₂-O]ₖ-R^{a},
   or R¹ and R², together with the atom to which they are attached, form a 5-membered heterocyclic ring optionally substituted with one or more substituents selected from C₁-C₆alkyl, C₂-C₆alkenyl, C₂-C₆alkynyl, (C₀-C₃alkylene)C₃-C₈cycloalkyl, (C₀-C₃alkylene)heterocycloalkyl, (C₀-C₃alkylene)C₆-C₁₄aryl, or (C₀-C₃alkylene)heteroaryl;
   R³ is a negative charge, H, or C₁-C₆ alkyl;
   R^{a} and R^{b} are independently, at each occurrence, H or C₁-C₆alkyl, wherein the alkyl is optionally substituted with one or more substituents selected from (C₀-C₃alkylene)C₃-C₈cycloalkyl, (C₀-C₃alkylene)heterocycloalkyl, (C₀-C₃alkylene)C₆-C₁₄aryl, or (C₀-C₃alkylene)heteroaryl; and
   k is an integer from 1 to 8,
   M¹ and M² are independently absent or a quaternary cation of Formula (II): wherein 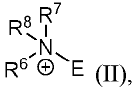
      E is C₁-C₆alkyl optionally substituted with one or more substituents selected from R⁴, R⁵, G-OR⁹, or (C₀-C₃alkylene)OR¹¹;
      R⁴ and R⁵ are independently, at each occurrence, H or C₁-C₆alkyl, C₂-C₆alkenyl, C₂-C₆alkynyl, (C₀-C₃alkylene)C₃-C₈cycloalkyl, (C₀-C₃alkylene)heterocycloalkyl, (C₀-C₃alkylene)C₆-C₁₄aryl, or (C₀-C₃alkylene)heteroaryl, wherein the alkyl, alkenyl, alkynyl, alkylene, cycloalkyl, heterocycloalkyl, aryl, or heteroaryl is optionally substituted with one or more substituents selected from cyano, halo, SeH, (C₀-C₃alkylene)NR^{c}R^{d}, (C₀-C₃alkylene)OR^{c}, (C₀-C₃alkylene)OC(O)R^{c}, (C₀-C₃alkylene)C(O)OR^{c}, (C₀-C₃alkylene)SR^{c}, (C₀-C₃alkylene)C(O)SR^{c}, (C₀-C₃alkylene)SC(O)R^{c}, (C₀-C₃alkylene)C(O)NR^{c}R^{d}, (C₀-C₃alkylene)NC(O)NR^{c}R^{d}, (C₀-C₃alkylene)C(NR^{c})NR^{c}R^{d}, (C₀-C₃alkylene)NR^{c}C(NR^{c})NR^{c}R^{d}, (C₀-C₃alkylene)P(O)OₙR^{c}R^{d}, (C₀-C₃alkylene)S(O)ₘNR^{c}R^{d}, (C₀-C₃alkylene)S(O)ₘOR^{c}, or (C₀-C₃alkylene)BOₚR^{c}R^{d};
      R⁶, R⁷, and R⁸, are independently H or C₁-C₆alkyl, wherein the alkyl is optionally substituted with one or more substituents selected from cyano, halo, (C₀-C₃alkylene)NR^{c}R^{d}, or (C₀-C₃alkylene)OR^{c},
      or R⁴ and R⁶, together with the atoms to which they are attached, form a 5- to 6-membered ring optionally substituted with one or more substituents selected from cyano, halo, (C₀-C₃alkylene)NR^{c}R^{d}, or (C₀-C₃alkylene)OR^{c},
      or R⁷ and R⁸, together with the atoms to which they are attached, form a 5- to 6-membered ring optionally substituted with one or more substituents selected from cyano, halo, (C₀-C₃alkylene)NR^{c}R^{d}, or (C₀-C₃alkylene)OR^{c};
      R^{c} and R^{d} are independently, at each occurrence, H or C₁-C₆alkyl, wherein the alkyl is optionally substituted with one or more substituents selected from (C₀-C₃alkylene)C₃-C₈cycloalkyl, (C₀-C₃alkylene)heterocycloalkyl, (C₀-C₃alkylene)C₆-C₁₄aryl, or (C₀-C₃alkylene)heteroaryl;
      R⁹ is a negative charge, H, or C₁-C₆ alkyl;
      R¹¹ is H, C₁-C₆alkyl, or C(O)C₁-C₆alkyl, wherein the C₁-C₆alkyl, or C(O)C₁-C₆alkyl is optionally substituted with one or more substituents selected from (C₀-C₃alkylene)NR^{c}R^{d}, (C₀-C₃alkylene)OR^{c}, (C₀-C₃alkylene)C₃-C₈cycloalkyl, (C₀-C₃alkylene)heterocycloalkyl, (C₀-C₃alkylene)C₆-C₁₄aryl, or (C₀-C₃alkylene)heteroaryl;
      G is CH₂ or C(O); and
      m, n, and p are independently, at each occurrence, 0, 1, or 2;
provided that
   a) at least one of M¹ or M² is a quaternary cation,
   b) when L is a bond, M¹ is absent, and
   c) when A is NR^{a}R^{b}, M² is absent.

Another aspect of the present disclosure relates to a pharmaceutical composition comprising a salt of Formula I, or a pharmaceutically acceptable salt thereof, in association with a pharmaceutically acceptable carrier.

Another aspect of the application relates to a salt of Formula (I), or enantiomer, stereoisomer, or tautomer thereof, for use in a method of treating infertility.

Another aspect of the present disclosure relates to a method of improving oocyte quality and maturation, comprising administering to a subject in need thereof, a therapeutically effective amount of a salt of Formula I.

In another aspect, a salt of Formula (I) is provided as a component in solution for use in treating a cell *ex vivo* for use in the treatment of an age related disorder. In some embodiments, the age related disorder is age-related infertility. In other aspects a salt of Formula (I) is provided as a component in solution for use in treating a cell *ex vivo* for use in the treatment of infertility.

Another aspect of present disclosure relates to a process for preparing salts of Formula (I), comprising contacting a nicotinic acid mononucleotide derivative of Formula II with a metal-alkali hydroxide under suitable conditions effective to produce the salt of Formula I.

In another aspect, the present disclosure relates to a cell culture medium for in vitro fertilization comprising: one or more salts of Formula (I) and culturing agents.

### Detailed Description of the Invention

The present application relates to salts and compositions that are capable of treating or preventing an age-related disorder. The salts and compositions of the present application can be used in methods of treating, preventing or ameliorating a disease or disorder associated with aging by administering to a patient in need thereof a therapeutically effective amount of a salt of Formula (I), or an enantiomer, stereoisomer, or tautomer thereof. The salts and compositions of the present application can be used in the treatment of a variety of diseases and disorders by preventing or ameliorating the process of aging and cellular restoration including, but not limited to, infertility, cellular degradation.

Salts of Formula (I) are potent and are efficacious at clinically achievable doses; are stable in a variety of potential dosage forms; possess acceptable solubility, acceptable pH, are crystalline, have a reduced propensity to absorb water, display ease of handling, - all of which are consistent with the development, manufacture and use of a medicament. In addition, the salts disclosed herein offer increased biological activity toward increased cellular NAD⁺ levels, increased stability and more physiologically acceptable pH.

A first aspect of disclosure relates to a salt of Formula I 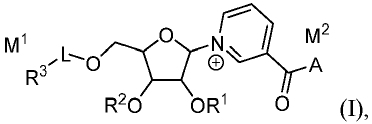 wherein A, L, M¹, M², R¹, R², and R³ are as described herein.

The articles "a" and "an" are used in this disclosure to refer to one or more than one (*i.e.,* to at least one) of the grammatical object of the article. By way of example, "an element" means one element or more than one element.

The term "and/or" is used in this disclosure to mean either "and" or "or" unless indicated otherwise.

The term "optionally substituted" is understood to mean that a given chemical moiety *(e.g.,* an alkyl group) can (but is not required to) be bonded other substituents (*e.g.,* heteroatoms). For instance, an alkyl group that is optionally substituted can be a fully saturated alkyl chain (*i.e.,* a pure hydrocarbon). Alternatively, the same optionally substituted alkyl group can have substituents different from hydrogen. For instance, it can, at any point along the chain be bound to a halogen atom, a hydroxyl group, or any other substituent described herein. Thus the term "optionally substituted" means that a given chemical moiety has the potential to contain other functional groups, but does not necessarily have any further functional groups. Suitable substituents used in the optional substitution of the described groups include, without limitation, halogen, oxo, -OH, -CN, -COOH, -CH₂CN, -O-(C₁-C₆) alkyl, (C₁-C₆) alkyl, C₁-C₆ alkoxy, (C₁-C₆)haloalkyl, C₁-C₆ haloalkoxy, -O-(C₂-C₆) alkenyl, -O-(C₂-C₆) alkynyl, (C₂-C₆) alkenyl, (C₂-C₆) alkynyl, -OH, -OP(O)(OH)₂, -OC(O)(C₁-C₆) alkyl, -C(O)(C₁-C₆)alkyl, -OC(O)O(C₁-C₆) alkyl, -NH₂, -NH((C₁-C₆) alkyl), -N((C₁-C₆) alkyl)₂, -NHC(O)(C₁-C₆) alkyl, -C(O)NH(C₁-C₆) alkyl, -S(O)₂(C₁-C₆) alkyl, -S(O)NH(C₁-C₆) alkyl, and S(O)N((C₁-C₆) alkyl)₂. The substituents can themselves be optionally substituted. "Optionally substituted" as used herein also refers to substituted or unsubstituted whose meaning is described below.

As used herein, the term "substituted" means that the specified group or moiety bears one or more suitable substituents wherein the substituents may connect to the specified group or moiety at one or more positions. For example, an aryl substituted with a cycloalkyl may indicate that the cycloalkyl connects to one atom of the aryl with a bond or by fusing with the aryl and sharing two or more common atoms.

As used herein, the term "unsubstituted" means that the specified group bears no substituents.

Unless otherwise specifically defined, the term "aryl" refers to cyclic, aromatic hydrocarbon groups that have 1 to 3 aromatic rings, including monocyclic or bicyclic groups such as phenyl, biphenyl or naphthyl. Where containing two aromatic rings (bicyclic, etc.), the aromatic rings of the aryl group may be joined at a single point (*e.g.,* biphenyl), or fused (*e.g.,* naphthyl). The aryl group may be optionally substituted by one or more substituents, e.g., 1 to 5 substituents, at any point of attachment. Exemplary substituents include, but are not limited to, -H, -halogen, - O-(C₁-C₆) alkyl, (C₁-C₆) alkyl, -O-(C₂-C₆) alkenyl, -O-(C₂-C₆) alkynyl, (C₂-C₆) alkenyl, (C₂-C₆) alkynyl, -OH, -OP(O)(OH)₂, -OC(O)(C₁-C₆) alkyl, -C(O)(C₁-C₆) alkyl, -OC(O)O(C₁-C₆) alkyl, NH₂, NH((C₁-C₆) alkyl), N((C₁-C₆) alkyl)₂, -S(O)₂-(C₁-C₆) alkyl, -S(O)NH(C₁-C₆) alkyl, and S(O)N((C₁-C₆) alkyl)₂. The substituents can themselves be optionally substituted. Furthermore when containing two fused rings the aryl groups herein defined may have an unsaturated or partially saturated ring fused with a fully saturated ring. Exemplary ring systems of these aryl groups include, but are not limited to, phenyl, biphenyl, naphthyl, anthracenyl, phenalenyl, phenanthrenyl, indanyl, indenyl, tetrahydronaphthalenyl, tetrahydrobenzoannulenyl, and the like.

Unless otherwise specifically defined, "heteroaryl" means a monovalent monocyclic aromatic radical of 5 to 24 ring atoms or a polycyclic aromatic radical, containing one or more ring heteroatoms selected from N, O, or S, the remaining ring atoms being C. Heteroaryl as herein defined also means a bicyclic heteroaromatic group wherein the heteroatom is selected from N, O, or S. The aromatic radical is optionally substituted independently with one or more substituents described herein. Examples include, but are not limited to, furyl, thienyl, pyrrolyl, pyridyl, pyrazolyl, pyrimidinyl, imidazolyl, isoxazolyl, oxazolyl, oxadiazolyl, pyrazinyl, indolyl, thiophen-2-yl, quinolyl, benzopyranyl, isothiazolyl, thiazolyl, thiadiazole, indazole, benzimidazolyl, thieno[3,2-b]thiophene, triazolyl, triazinyl, imidazo[1,2-b]pyrazolyl, furo[2,3-c]pyridinyl, imidazo[1,2-a]pyridinyl, indazolyl, pyrrolo[2,3-c]pyridinyl, pyrrolo[3,2-c]pyridinyl, pyrazolo[3,4-c]pyridinyl, thieno[3,2-c]pyridinyl, thieno[2,3-c]pyridinyl, thieno[2,3-b]pyridinyl, benzothiazolyl, indolyl, indolinyl, indolinonyl, dihydrobenzothiophenyl, dihydrobenzofuranyl, benzofuran, chromanyl, thiochromanyl, tetrahydroquinolinyl, dihydrobenzothiazine, dihydrobenzoxanyl, quinolinyl, isoquinolinyl, 1,6-naphthyridinyl, benzo[de]isoquinolinyl, pyrido[4,3-b][1,6]naphthyridinyl, thieno[2,3-b]pyrazinyl, quinazolinyl, tetrazolo[1,5-a]pyridinyl, [1,2,4]triazolo[4,3-a]pyridinyl, isoindolyl, pyrrolo[2,3-b]pyridinyl, pyrrolo[3,4-b]pyridinyl, pyrrolo[3,2-b]pyridinyl, imidazo[5,4-b]pyridinyl, pyrrolo[1,2-a]pyrimidinyl, tetrahydro pyrrolo[1,2-a]pyrimidinyl, 3,4-dihydro-2H-1λ²-pyrrolo[2,1-b]pyrimidine, dibenzo[b,d] thiophene, pyridin-2-one, furo[3,2-c]pyridinyl, furo[2,3-c]pyridinyl, 1H-pyrido[3,4-b][1,4] thiazinyl, benzooxazolyl, benzoisoxazolyl, furo[2,3-b]pyridinyl, benzothiophenyl, 1,5-naphthyridinyl, furo[3,2-b]pyridine, [1,2,4]triazolo[1,5-a]pyridinyl, benzo [1,2,3]triazolyl, imidazo[1,2-a]pyrimidinyl, [1,2,4]triazolo[4,3-b]pyridazinyl, benzo[c][1,2,5]thiadiazolyl, benzo[c][1,2,5]oxadiazole, 1,3-dihydro-2H-benzo[d]imidazol-2-one, 3,4-dihydro-2H-pyrazolo [1,5-b][1,2]oxazinyl, 4,5,6,7-tetrahydropyrazolo[1,5-a]pyridinyl, thiazolo[5,4-d]thiazolyl, imidazo[2,1-b][1,3,4]thiadiazolyl, thieno[2,3-b]pyrrolyl, 3H-indolyl, and derivatives thereof. Furthermore when containing two fused rings the aryl groups herein defined may have an unsaturated or partially saturated ring fused with a fully saturated ring. Exemplary ring systems of these heteroaryl groups include indolinyl, indolinonyl, dihydrobenzothiophenyl, dihydrobenzofuran, chromanyl, thiochromanyl, tetrahydroquinolinyl, dihydrobenzothiazine, 3,4-dihydro-1H-isoquinolinyl, 2,3-dihydrobenzofuran, indolinyl, indolyl, and dihydrobenzoxanyl.

Halogen or "halo" refers to fluorine, chlorine, bromine, or iodine.

"Alkyl" refers to a straight or branched chain saturated hydrocarbon containing 1-12 carbon atoms. Examples of a (C₁-C₆) alkyl group include, but are not limited to, methyl, ethyl, propyl, butyl, pentyl, hexyl, isopropyl, isobutyl, sec-butyl, tert-butyl, isopentyl, neopentyl, and isohexyl.

"Alkoxy" refers to a straight or branched chain saturated hydrocarbon containing 1-12 carbon atoms containing a terminal "O" in the chain, *i.e.,* -O(alkyl). Examples of alkoxy groups include, without limitation, methoxy, ethoxy, propoxy, butoxy, t-butoxy, or pentoxy groups.

"Alkenyl" refers to a straight or branched chain unsaturated hydrocarbon containing 2-12 carbon atoms. The "alkenyl" group contains at least one double bond in the chain. The double bond of an alkenyl group can be unconjugated or conjugated to another unsaturated group. Examples of alkenyl groups include ethenyl, propenyl, n-butenyl, iso-butenyl, pentenyl, or hexenyl. An alkenyl group can be unsubstituted or substituted. Alkenyl, as herein defined, may be straight or branched.

"Alkynyl" refers to a straight or branched chain unsaturated hydrocarbon containing 2-12 carbon atoms. The "alkynyl" group contains at least one triple bond in the chain. Examples of alkenyl groups include ethynyl, propargyl, n-butynyl, iso-butynyl, pentynyl, or hexynyl. An alkynyl group can be unsubstituted or substituted.

The term "alkylene" or "alkylenyl" refers to a divalent alkyl radical. Any of the above mentioned monovalent alkyl groups may be an alkylene by abstraction of a second hydrogen atom from the alkyl. As herein defined, alkylene may also be a C₁-C₆ alkylene. An alkylene may further be a C₁-C₄alkylene. Typical alkylene groups include, but are not limited to, -CH₂-, -CH(CH₃)-, - C(CH₃)₂-, -CH₂CH₂-, -CH₂CH(CH₃)-, -CH₂C(CH₃)₂-, -CH₂CH₂CH₂-, -CH₂CH₂CH₂CH₂-, and the like.

"Cycloalkyl" means monocyclic or polycyclic saturated carbon rings (*e.g.,* fused, bridged, or spiro rings) containing 3-18 carbon atoms (*e.g*., C₃-C₁₀). Examples of cycloalkyl groups include, without limitations, cyclopropyl, cyclobutyl, cyclopentyl, cyclohexyl, cycloheptanyl, cyclooctanyl, norboranyl, norborenyl, bicyclo[2.2.2]octanyl, or bicyclo[2.2.2]octenyl.

"Heterocyclyl" or "heterocycloalkyl" means monocyclic or polycyclic rings (*e.g.,* fused, bridged, or spiro rings) containing carbon and heteroatoms taken from oxygen, nitrogen, or sulfur and wherein there is not delocalized π electrons (aromaticity) shared among the ring carbon or heteroatoms. The heterocycloalkyl can be a 3-, 4-, 5-, 6-, 7-, 8-, 9- 10-, 11-, or 12-membered ring. The heterocycloalkyl ring structure may be substituted by one or more substituents. The substituents can themselves be optionally substituted. Examples of heterocyclyl rings include, but are not limited to, oxetanyl, azetadinyl, tetrahydrofuranyl, tetrahydropyranyl, pyrrolidinyl, oxazolinyl, oxazolidinyl, thiazolinyl, thiazolidinyl, pyranyl, thiopyranyl, tetrahydropyranyl, dioxalinyl, piperidinyl, morpholinyl, thiomorpholinyl, thiomorpholinyl S-oxide, thiomorpholinyl S-dioxide, piperazinyl, azepinyl, oxepinyl, diazepinyl, tropanyl, oxazolidinonyl, and homotropanyl. In accordance with the present application, 3- to 10- membered heterocyclyl refers to saturated or partially saturated non-aromatic rings structures containing between 3 and 10 atoms in which there is at least one heteroatoms selected from the group N, O, or S.

The term "hydroxyalkyl" means an alkyl group as defined above, where the alkyl group is substituted with one or more -OH groups. Examples of hydroxyalkyl groups include HO-CH₂- , HO-CH₂-CH₂- and CH₃-CH(OH)-.

The term "haloalkyl" as used herein refers to an alkyl group, as defined herein, which is substituted one or more halogen. Examples of haloalkyl groups include, but are not limited to, trifluoromethyl, difluoromethyl, pentafluoroethyl, trichloromethyl, etc.

The term "haloalkoxy" as used herein refers to an alkoxy group, as defined herein, which is substituted one or more halogen. Examples of haloalkyl groups include, but are not limited to, trifluoromethoxy, difluoromethoxy, pentafluoroethoxy, trichloromethoxy, etc.

The term "cyano" as used herein means a substituent having a carbon atom joined to a nitrogen atom by a triple bond, *i.e.,* C≡N.

The term "amine" as used herein refers to primary (R-NH₂, R ≠ H), secondary (R₂-NH, R₂≠ H) and tertiary (R₃-N, R ≠ H) amines. A substituted amine is intended to mean an amine where at least one of the hydrogen atoms has been replaced by the substituent.

The term "amino" as used herein means a substituent containing at least one nitrogen atom. Specifically, NH₂, -NH(alkyl) or alkylamino, -N(alkyl)₂ or dialkylamino, amide-, carbamide-, urea, and sulfamide substituents are included in the term "amino".

The term "oxo" as used herein refers to an "=O" group.

The term "quaternary cation" means a quaternary ammonium or phosphonium ion having on the nitrogen or phosphorus atom thereof four substituents which may be identical or different. Specific examples of said quaternary cations include trimethylglycine, carnitine, choline, or the like.

The term "isomer" refers to salts and/or compounds that have the same composition and molecular weight but differ in physical and/or chemical properties. The structural difference may be in constitution (geometric isomers) or in the ability to rotate the plane of polarized light (stereoisomers). With regard to stereoisomers, the salts of Formula (I) may have one or more asymmetric carbon atom and may occur as racemates, racemic mixtures and as individual enantiomers or diastereomers.

The disclosure also includes pharmaceutical compositions comprising an effective amount of a disclosed salt and a pharmaceutically acceptable carrier. Representative "pharmaceutically acceptable salts" include, e.g., water-soluble and water-insoluble salts, such as the acetate, amsonate (4,4-diaminostilbene-2,2-disulfonate), benzenesulfonate, benzonate, bicarbonate, bisulfate, bitartrate, borate, bromide, butyrate, calcium, calcium edetate, camsylate, carbonate, chloride, citrate, clavulariate, dihydrochloride, edetate, edisylate, estolate, esylate, fumerate, fiunarate, gluceptate, gluconate, glutamate, glycollylarsanilate, hexafluorophosphate, hexylresorcinate, hydrabamine, hydrobromide, hydrochloride, hydroxynaphthoate, iodide, isothionate, lactate, lactobionate, laurate, magnesium, malate, maleate, mandelate, mesylate, methylbromide, methylnitrate, methylsulfate, mucate, napsylate, nitrate, N-methylglucamine ammonium salt, 3-hydroxy-2-naphthoate, oleate, oxalate, palmitate, pamoate (1,1-methene-bis-2-hydroxy-3-naphthoate, einbonate), pantothenate, phosphate/diphosphate, picrate, polygalacturonate, propionate, p-toluenesulfonate, salicylate, stearate, subacetate, succinate, sulfate, sulfosalicylate, suramate, tannate, tartrate, teoclate, tosylate, triethiodide, and valerate salts.

A "patient" or "subject" is a mammal, e.g., a human, mouse, rat, guinea pig, dog, cat, horse, cow, pig, or non-human primate, such as a monkey, chimpanzee, baboon or rhesus.

An "effective amount" when used in connection with a salt or pharmaceutical composition is an amount effective for treating or preventing a disease in a subject as described herein.

The term "carrier", as used in this disclosure, encompasses carriers, excipients, and diluents and means a material, composition or vehicle, such as a liquid or solid filler, diluent, excipient, solvent or encapsulating material, involved in carrying or transporting a pharmaceutical agent from one organ, or portion of the body, to another organ, or portion of the body of a subject.

The term "treating" with regard to a subject, refers to improving at least one symptom of the subject's disorder. Treating includes curing, improving, or at least partially ameliorating the disorder.

The term "disorder" is used in this disclosure to mean, and is used interchangeably with, the terms disease, condition, or illness, unless otherwise indicated.

The term "administer", "administering", or "administration" as used in this disclosure refers to either directly administering a disclosed salt or a composition to a subject, or administering a prodrug derivative or analog of the salt or composition to the subject, which can form an equivalent amount of active salt within the subject's body.

### Salts of the application

The present application relates to salts or enantiomers, stereoisomers, or tautomers thereof, capable of treating or preventing an age-related disorder, which are useful for the treatment of diseases and disorders associated with aging and cellular restoration.

In one embodiment of the salt of Formula I, A is O⁻. In another embodiment, A is NR^{a}R^{b}.

In one embodiment of the salt of Formula I, L is a bond. In another embodiment, L is

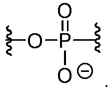

**In** some embodiments of the invention, R^{a} is independently, at each occurrence H, or C₁-C₆alkyl. In other embodiments, R^{a} is H. In other embodiments, R^{a} is C₁-C₆alkyl. In other embodiments, R^{a} is C₁-C₆alkyl substituted with one or more substituents selected from C₁-C₆alkyl, C₂-C₆alkenyl, C₂-C₆alkynyl, (C₀-C₃alkylene)C₃-C₈cycloalkyl, (C₀-C₃alkylene)heterocycloalkyl, (C₀-C₃alkylene)C₆-C₁₄aryl, or (C₀-C₃alkylene)heteroaryl. In other embodiments, R^{a} is C₁-C₆alkyl substituted with one or more substituents selected from C₁-C₆alkyl. In other embodiments, R^{a} is C₁-C₆alkyl substituted with one or C₂-C₆alkenyl. In other embodiments, R^{a} is C₁-C₆alkyl substituted with one or more C₂-C₆alkynyl. In other embodiments, R^{a} is C₁-C₆alkyl substituted with one or more m (C₀-C₃alkylene)C₃-C₈cycloalkyl. In other embodiments, R^{a} is C₁-C₆alkyl substituted with one or more (C₀-C₃alkylene)heterocycloalkyl. In other embodiments, R^{a} is C₁-C₆alkyl substituted with one or more (C₀-C₃alkylene)C₆-C₁₄aryl. In other embodiments, R^{a} is C₁-C₆alkyl substituted with one or more (C₀-C₃alkylene)heteroaryl. In other embodiment R^{a} is methyl. In other embodiment R^{a} is methyl substituted with one or more substituents selected from C₁-C₆alkyl, C₂-C₆alkenyl, C₂-C₆alkynyl, (C₀-C₃alkylene)C₃-C₈cycloalkyl, (C₀-C₃alkylene)heterocycloalkyl, (C₀-C₃alkylene)C₆-C₁₄aryl, or (C₀-C₃alkylene)heteroaryl.

In some embodiments of the invention, R^{b} is independently, at each occurrence H, or C₁-C₆alkyl. In other embodiments, R^{b} is H. In other embodiments, R^{b} is C₁-C₆alkyl. In other embodiments, R^{b} is C₁-C₆alkyl substituted with one or more substituents selected from C₁-C₆alkyl, C₂-C₆alkenyl, C₂-C₆alkynyl, (C₀-C₃alkylene)C₃-C₈cycloalkyl, (C₀-C₃alkylene)heterocycloalkyl, (C₀-C₃alkylene)C₆-C₁₄aryl, or (C₀-C₃alkylene)heteroaryl. In other embodiments, R^{b} is C₁-C₆alkyl substituted with one or more substituents selected from C₁-C₆alkyl. In other embodiments, R^{b} is C₁-C₆alkyl substituted with one or C₂-C₆alkenyl. In other embodiments, R^{b} is C₁-C₆alkyl substituted with one or more C₂-C₆alkynyl. In other embodiments, R^{b} is C₁-C₆alkyl substituted with one or more m (C₀-C₃alkylene)C₃-C₈cycloalkyl. In other embodiments, R^{b} is C₁-C₆alkyl substituted with one or more (C₀-C₃alkylene)heterocycloalkyl. In other embodiments, R^{b} is C₁-C₆alkyl substituted with one or more (C₀-C₃alkylene)C₆-C₁₄aryl. In other embodiments, R^{b} is C₁-C₆alkyl substituted with one or more (C₀-C₃alkylene)heteroaryl. In other embodiment R^{b} is methyl. In other embodiment R^{b} is methyl substituted with one or more substituents selected from C₁-C₆alkyl, C₂-C₆alkenyl, C₂-C₆alkynyl, (C₀-C₃alkylene)C₃-C₈cycloalkyl, (C₀-C₃alkylene)heterocycloalkyl, (C₀-C₃alkylene)C₆-C₁₄aryl, or (C₀-C₃alkylene)heteroaryl.

Yet in another embodiment, R³ is a negative charge, H, or C₁-C₆ alkyl. In one embodiment, R³ represents a negative charge. In another embodiment, R³ is H. In another embodiment, R³ is C₁-C₆ alkyl.

In a further embodiment, R¹ is independently H, C₁-C₆alkyl, C₁-C₆haloalkyl, - C(O)C₁-C₆haloalkyl, (C₀-C₃alkylene)C(O)C₁-C₆alkyl, -C(O)OR^{a}, -C(O)NR^{a}R^{b},-[CH₂-CH₂-O]ₖ-R^{a}, -C(O)[CH₂-CH₂-O]ₖ-R^{a}, -[CH₂-CH₂-CH₂-O]ₖ-R^{a}, or -C(O)[CH₂-CH₂-CH₂-O]ₖ-R^{a}. In another embodiment, R¹ is H. In another embodiment, R¹ is C₁-C₆alkyl. In another embodiment, R¹ is C₁-C₆haloalkyl. In another embodiment, R¹ is (C₀-C₃alkylene)C(O)C₁-C₆alkyl. In another embodiment, R¹ is -C(O)OR^{a}. In another embodiment, R¹ is -[CH₂-CH₂-O]ₖ-R^{a}. In another embodiment, R¹ is C(O)C₁-C₆alkyl. In another embodiment, R¹ is -C(O)C₁-C₆haloalkyl. In another embodiment, R¹ is -C(O)[CH₂-CH₂-O]ₖ-R^{a}. In another embodiment, R¹ is-[CH₂-CH₂-CH₂-O]ₖ-R^{a}. In another embodiment, R¹ is -C(O)[CH₂-CH₂-CH₂-O]ₖ-R^{a}.

In one embodiment, R² is independently H, C₁-C₆alkyl, C₁-C₆haloalkyl, -C(O)C₁-C₆haloalkyl, (C₀-C₃alkylene)C(O)C₁-C₆alkyl, -C(O)OR^{a}, -C(O)NR^{a}R^{b},-[CH₂-CH₂-O]ₖ-R^{a}, - C(O)[CH₂-CH₂-O]ₖ-R^{a}, -[CH₂-CH₂-CH₂-O]ₖ-R^{a}, or -C(O)[CH₂-CH₂-CH₂-O]ₖ-R^{a}. In another embodiment, R² is H. In another embodiment, R² is C₁-C₆alkyl. In another embodiment, R² is C₁-C₆haloalkyl. In another embodiment, R² is (C₀-C₃alkylene)C(O)C₁-C₆alkyl. In another embodiment, R² is -C(O)OR^{a}. In another embodiment, R² is -[CH₂-CH₂-O]ₖ-R^{a}. In another embodiment, R² is C(O)C₁-C₆alkyl. In another embodiment, R² is -C(O)C₁-C₆haloalkyl. In another embodiment, R² is -C(O)[CH₂-CH₂-O]ₖ-R^{a}. In another embodiment, R² is-[CH₂-CH₂-CH₂-O]ₖ-R^{a}. In another embodiment, R² is -C(O)[CH₂-CH₂-CH₂-O]ₖ-R^{a}.

In a further embodiment of the salts of the Formula I, R¹ and R², together with the atom to which they are attached, may form a 5-membered heterocyclic ring. In a further embodiment of the salts of the Formula I, R¹ and R², together with the atom to which they are attached, may form a 6-membered heterocyclic ring. In yet a further embodiment of the salts of the Formula I, R¹ and R², together with the atom to which they are attached, may form a 5-membered heterocyclic ring substituted with one or more substituents selected from C₁-C₆alkyl, C₂-C₆alkenyl, C₂-C₆alkynyl, (C₀-C₃alkylene)C₃-C₈cycloalkyl, (C₀-C₃alkylene)heterocycloalkyl, (C₀-C₃alkylene)C₆-C₁₄aryl, and (C₀-C₃alkylene)heteroaryl. In yet a further embodiment of the salts of the Formula I, R¹ and R², together with the atom to which they are attached, may form a 6-membered heterocyclic ring substituted with one or more substituents selected from C₁-C₆alkyl, C₂-C₆alkenyl, C₂-C₆alkynyl, (C₀-C₃alkylene)C₃-C₈cycloalkyl, (C₀-C₃alkylene)heterocycloalkyl, (C₀-C₃alkylene)C₆-C₁₄aryl, and (C₀-C₃alkylene)heteroaryl.

In one embodiment of the salt of Formula I, M¹ is absent. In another embodiment, M¹ is a quaternary cation of Formula II:

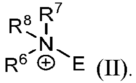

In one embodiment of the salt of Formula I, M² is absent. In another embodiment, M² is a quaternary cation of Formula II:

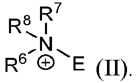

In one embodiment of the salt of Formula I, M¹ is absent and M² is a quaternary cation of Formula II:

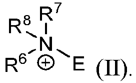

In one embodiment of the salt of Formula I, M² is absent and M¹ is a quaternary cation of Formula II:

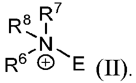

In one embodiment of the salt of Formula I, M¹ and M² are each a quaternary cation of Formula II:

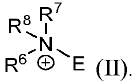

In one embodiment of the salt of Formula I, M¹ is a quaternary cation of Formula IIa: 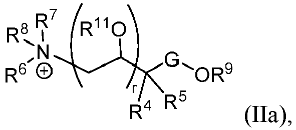 wherein r is 0 or 1.

In one embodiment of the salt of Formula I, M² is a quaternary cation of Formula IIa: 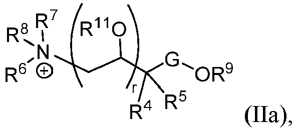 wherein r is 0 or 1.

In one embodiment of the salt of Formula I, M¹ is 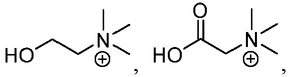 or 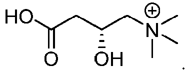 In another embodiment, M¹ is 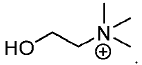 In another embodiment, M¹ is 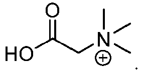 In another embodiment, M¹ is

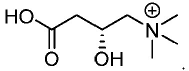

In one embodiment of the salt of Formula I, M¹ and M² are 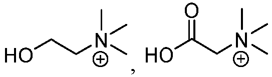 , or 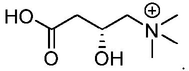 In another embodiment, M¹ and M² are 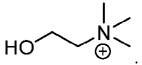 In another embodiment, M¹ and M² are 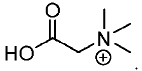 In another embodiment, M¹ and M² are

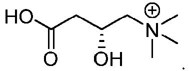

In one embodiment of the salt of Formula I, R⁴ is H, C₁-C₆alkyl, C₂-C₆alkenyl, C₂-C₆alkynyl, (C₀-C₃alkylene)C₃-C₈cycloalkyl, (C₀-C₃alkylene)heterocycloalkyl, (C₀-C₃alkylene)C₆-C₁₄aryl, or (C₀-C₃alkylene)heteroaryl. In another embodiment, R⁴ is H. In another embodiment, R⁴ is C₁-C₆alkyl. In another embodiment, R⁴ is C₂-C₆alkenyl. In another embodiment, R⁴ is C₂-C₆alkynyl. In another embodiment, R⁴ is (C₀-C₃alkylene)C₃-C₈cycloalkyl. In another embodiment, R⁴ is (C₀-C₃alkylene)heterocycloalkyl. In another embodiment, R⁴ is (C₀-C₃alkylene)C₆-C₁₄aryl. In another embodiment, R⁴ is (C₀-C₃alkylene)heteroaryl. In another embodiment, R⁴ is C₁-C₄alkyl substituted with one or more (C₀-C₃alkylene)SR^{c}.

In another embodiment, R⁴ is C₁-C₆alkyl substituted with one or more substituents selected from cyano, halo, SeH, (C₀-C₃alkylene)NR^{c}R^{d}, (C₀-C₃alkylene)OR^{c}, (C₀-C₃alkylene)OC(O)R^{c}, (C₀-C₃alkylene)C(O)OR^{c}, (C₀-C₃alkylene)SR^{c}, (C₀-C₃alkylene)C(O)SR^{c}, (C₀-C₃alkylene)SC(O)R^{c}, (C₀-C₃alkylene)C(O)NR^{c}R^{d}, (C₀-C₃alkylene)NC(O)NR^{c}R^{d}, (C₀-C₃alkylene)C(NR^{c})NR^{c}R^{d}, (C₀-C₃alkylene)NR^{c}C(NR^{c})NR^{c}R^{d}, (C₀-C₃alkylene)P(O)OₙR^{c}R^{d}, (C₀-C₃alkylene)S(O)ₘNR^{c}R^{d}, (C₀-C₃alkylene)S(O)ₘOR^{c}, or (C₀-C₃alkylene)BOₚR^{c}R^{d}. In another embodiment, R⁴ is C₂-C₆alkenyl substituted with one or more substituents selected from cyano, halo, SeH, (C₀-C₃alkylene)NR^{c}R^{d}, (C₀-C₃alkylene)OR^{c}, (C₀-C₃alkylene)OC(O)R^{c}, (C₀-C₃alkylene)C(O)OR^{c}, (C₀-C₃alkylene)SR^{c}, (C₀-C₃alkylene)C(O)SR^{c}, (C₀-C₃alkylene)SC(O)R^{c}, (C₀-C₃alkylene)C(O)NR^{c}R^{d}, (C₀-C₃alkylene)NC(O)NR^{c}R^{d}, (C₀-C₃alkylene)C(NR^{c})NR^{c}R^{d}, (C₀-C₃alkylene)NR^{c}C(NR^{c})NR^{c}R^{d}, (C₀-C₃alkylene)P(O)OₙR^{c}R^{d}, (C₀-C₃alkylene)S(O)ₘNR^{c}R^{d}, (C₀-C₃alkylene)S(O)ₘOR^{c}, or (C₀-C₃alkylene)BOₚR^{c}R^{d}. In another embodiment, R⁴ is C₂-C₆alkynyl substituted with one or more substituents selected from cyano, halo, SeH, (C₀-C₃alkylene)NR^{c}R^{d}, (C₀-C₃alkylene)OR^{c}, (C₀-C₃alkylene)OC(O)R^{c}, (C₀-C₃alkylene)C(O)OR^{c}, (C₀-C₃alkylene)SR^{c}, (C₀-C₃alkylene)C(O)SR^{c}, (C₀-C₃alkylene)SC(O)R^{c}, (C₀-C₃alkylene)C(O)NR^{c}R^{d}, (C₀-C₃alkylene)NC(O)NR^{c}R^{d}, (C₀-C₃alkylene)C(NR^{c})NR^{c}R^{d}, (C₀-C₃alkylene)NR^{c}C(NR^{c})NR^{c}R^{d}, (C₀-C₃alkylene)P(O)OₙR^{c}R^{d}, (C₀-C₃alkylene)S(O)ₘNR^{c}R^{d}, (C₀-C₃alkylene)S(O)ₘOR^{c}, or (C₀-C₃alkylene)BOₚR^{c}R^{d}. In another embodiment, R⁴ is (C₀-C₃alkylene)C₃-C₈cycloalkyl substituted with one or more substituents selected from cyano, halo, SeH, (C₀-C₃alkylene)NR^{c}R^{d}, (C₀-C₃alkylene)OR^{c}, (C₀-C₃alkylene)OC(O)R^{c}, (C₀-C₃alkylene)C(O)OR^{c}, (C₀-C₃alkylene)SR^{c}, (C₀-C₃alkylene)C(O)SR^{c}, (C₀-C₃alkylene)SC(O)R^{c}, (C₀-C₃alkylene)C(O)NR^{c}R^{d}, (C₀-C₃alkylene)NC(O)NR^{c}R^{d}, (C₀-C₃alkylene)C(NR^{c})NR^{c}R^{d}, (C₀-C₃alkylene)NR^{c}C(NR^{c})NR^{c}R^{d}, (C₀-C₃alkylene)P(O)OₙR^{c}R^{d}, (C₀-C₃alkylene)S(O)ₘNR^{c}R^{d}, (C₀-C₃alkylene)S(O)ₘOR^{c}, or (C₀-C₃alkylene)BOₚR^{c}R^{d}. In another embodiment, R⁴ is (C₀-C₃alkylene)heterocycloalkyl substituted with one or more substituents selected from cyano, halo, SeH, (C₀-C₃alkylene)NR^{c}R^{d}, (C₀-C₃alkylene)OR^{c}, (C₀-C₃alkylene)OC(O)R^{c}, (C₀-C₃alkylene)C(O)OR^{c}, (C₀-C₃alkylene)SR^{c}, (C₀-C₃alkylene)C(O)SR^{c}, (C₀-C₃alkylene)SC(O)R^{c}, (C₀-C₃alkylene)C(O)NR^{c}R^{d}, (C₀-C₃alkylene)NC(O)NR^{c}R^{d}, (C₀-C₃alkylene)C(NR^{c})NR^{c}R^{d}, (C₀-C₃alkylene)NR^{c}C(NR^{c})NR^{c}R^{d}, (C₀-C₃alkylene)P(O)OₙR^{c}R^{d}, (C₀-C₃alkylene)S(O)ₘNR^{c}R^{d}, (C₀-C₃alkylene)S(O)ₘOR^{c}, or (C₀-C₃alkylene)BOₚR^{c}R^{d}. In another embodiment, R⁴ is (C₀-C₃alkylene)C₆-C₁₄aryl substituted with one or more substituents selected from cyano, halo, SeH, (C₀-C₃alkylene)NR^{c}R^{d}, (C₀-C₃alkylene)OR^{c}, (C₀-C₃alkylene)OC(O)R^{c}, (C₀-C₃alkylene)C(O)OR^{c}, (C₀-C₃alkylene)SR^{c}, (C₀-C₃alkylene)C(O)SR^{c}, (C₀-C₃alkylene)SC(O)R^{c}, (C₀-C₃alkylene)C(O)NR^{c}R^{d}, (C₀-C₃alkylene)NC(O)NR^{c}R^{d}, (C₀-C₃alkylene)C(NR^{c})NR^{c}R^{d}, (C₀-C₃alkylene)NR^{c}C(NR^{c})NR^{c}R^{d}, (C₀-C₃alkylene)P(O)OₙR^{c}R^{d}, (C₀-C₃alkylene)S(O)ₘNR^{c}R^{d}, (C₀-C₃alkylene)S(O)ₘOR^{c}, or (C₀-C₃alkylene)BOₚR^{c}R^{d}. In another embodiment, R⁴ is (C₀-C₃alkylene)heteroaryl substituted with one or more substituents selected from cyano, halo, SeH, (C₀-C₃alkylene)NR^{c}R^{d}, (C₀-C₃alkylene)OR^{c}, (C₀-C₃alkylene)OC(O)R^{c}, (C₀-C₃alkylene)C(O)OR^{c}, (C₀-C₃alkylene)SR^{c}, (C₀-C₃alkylene)C(O)SR^{c}, (C₀-C₃alkylene)SC(O)R^{c}, (C₀-C₃alkylene)C(O)NR^{c}R^{d}, (C₀-C₃alkylene)NC(O)NR^{c}R^{d}, (C₀-C₃alkylene)C(NR^{c})NR^{c}R^{d}, (C₀-C₃alkylene)NR^{c}C(NR^{c})NR^{c}R^{d}, (C₀-C₃alkylene)P(O)OₙR^{c}R^{d}, (C₀-C₃alkylene)S(O)ₘNR^{c}R^{d}, (C₀-C₃alkylene)S(O)ₘOR^{c}, or (C₀-C₃alkylene)BOₚR^{c}R^{d}.

In one embodiment of the salt of Formula I, R⁵ is H, C₁-C₆alkyl, C₂-C₆alkenyl, C₂-C₆alkynyl, (C₀-C₃alkylene)C₃-C₈cycloalkyl, (C₀-C₃alkylene)heterocycloalkyl, (C₀-C₃alkylene)C₆-C₁₄aryl, or (C₀-C₃alkylene)heteroaryl. In another embodiment, R⁵ is H. In another embodiment, R⁵ is C₁-C₆alkyl. In another embodiment, R⁵ is C₂-C₆alkenyl. In another embodiment, R⁵ is C₂-C₆alkynyl. In another embodiment, R⁵ is (C₀-C₃alkylene)C₃-C₈cycloalkyl. In another embodiment, R⁵ is (C₀-C₃alkylene)heterocycloalkyl. In another embodiment, R⁵ is (C₀-C₃alkylene)C₆-C₁₄aryl. In another embodiment, R⁵ is (C₀-C₃alkylene)heteroaryl.

In another embodiment, R⁵ is C₁-C₆alkyl substituted with one or more substituents selected from cyano, halo, SeH, (C₀-C₃alkylene)NR^{c}R^{d}, (C₀-C₃alkylene)OR^{c}, (C₀-C₃alkylene)OC(O)R^{c}, (C₀-C₃alkylene)C(O)OR^{c}, (C₀-C₃alkylene)SR^{c}, (C₀-C₃alkylene)C(O)SR^{c}, (C₀-C₃alkylene)SC(O)R^{c}, (C₀-C₃alkylene)C(O)NR^{c}R^{d}, (C₀-C₃alkylene)NC(O)NR^{c}R^{d}, (C₀-C₃alkylene)C(NR^{c})NR^{c}R^{d}, (C₀-C₃alkylene)NR^{c}C(NR^{c})NR^{c}R^{d}, (C₀-C₃alkylene)P(O)OₙR^{c}R^{d}, (C₀-C₃alkylene)S(O)ₘNR^{c}R^{d}, (C₀-C₃alkylene)S(O)ₘOR^{c}, or (C₀-C₃alkylene)BOₚR^{c}R^{d}. In another embodiment, R⁵ is C₂-C₆alkenyl substituted with one or more substituents selected from cyano, halo, SeH, (C₀-C₃alkylene)NR^{c}R^{d}, (C₀-C₃alkylene)OR^{c}, (C₀-C₃alkylene)OC(O)R^{c}, (C₀-C₃alkylene)C(O)OR^{c}, (C₀-C₃alkylene)SR^{c}, (C₀-C₃alkylene)C(O)SR^{c}, (C₀-C₃alkylene)SC(O)R^{c}, (C₀-C₃alkylene)C(O)NR^{c}R^{d}, (C₀-C₃alkylene)NC(O)NR^{c}R^{d}, (C₀-C₃alkylene)C(NR^{c})NR^{c}R^{d}, (C₀-C₃alkylene)NR^{c}C(NR^{c})NR^{c}R^{d}, (C₀-C₃alkylene)P(O)OₙR^{c}R^{d}, (C₀-C₃alkylene)S(O)ₘNR^{c}R^{d}, (C₀-C₃alkylene)S(O)ₘOR^{c}, or (C₀-C₃alkylene)BOₚR^{c}R^{d}. In another embodiment, R⁵ is C₂-C₆alkynyl substituted with one or more substituents selected from cyano, halo, SeH, (C₀-C₃alkylene)NR^{c}R^{d}, (C₀-C₃alkylene)OR^{c}, (C₀-C₃alkylene)OC(O)R^{c}, (C₀-C₃alkylene)C(O)OR^{c}, (C₀-C₃alkylene)SR^{c}, (C₀-C₃alkylene)C(O)SR^{c}, (C₀-C₃alkylene)SC(O)R^{c}, (C₀-C₃alkylene)C(O)NR^{c}R^{d}, (C₀-C₃alkylene)NC(O)NR^{c}R^{d}, (C₀-C₃alkylene)C(NR^{c})NR^{c}R^{d}, (C₀-C₃alkylene)NR^{c}C(NR^{c})NR^{c}R^{d}, (C₀-C₃alkylene)P(O)OₙR^{c}R^{d}, (C₀-C₃alkylene)S(O)ₘNR^{c}R^{d}, (C₀-C₃alkylene)S(O)ₘOR^{c}, or (C₀-C₃alkylene)BOₚR^{c}R^{d}. In another embodiment, R⁵ is (C₀-C₃alkylene)C₃-C₈cycloalkyl substituted with one or more substituents selected from cyano, halo, SeH, (C₀-C₃alkylene)NR^{c}R^{d}, (C₀-C₃alkylene)OR^{c}, (C₀-C₃alkylene)OC(O)R^{c}, (C₀-C₃alkylene)C(O)OR^{c}, (C₀-C₃alkylene)SR^{c}, (C₀-C₃alkylene)C(O)SR^{c}, (C₀-C₃alkylene)SC(O)R^{c}, (C₀-C₃alkylene)C(O)NR^{c}R^{d}, (C₀-C₃alkylene)NC(O)NR^{c}R^{d}, (C₀-C₃alkylene)C(NR^{c})NR^{c}R^{d}, (C₀-C₃alkylene)NR^{c}C(NR^{c})NR^{c}R^{d}, (C₀-C₃alkylene)P(O)OₙR^{c}R^{d}, (C₀-C₃alkylene)S(O)ₘNR^{c}R^{d}, (C₀-C₃alkylene)S(O)ₘOR^{c}, or (C₀-C₃alkylene)BOₚR^{c}R^{d}. In another embodiment, R⁵ is (C₀-C₃alkylene)heterocycloalkyl substituted with one or more substituents selected from cyano, halo, SeH, (C₀-C₃alkylene)NR^{c}R^{d}, (C₀-C₃alkylene)OR^{c}, (C₀-C₃alkylene)OC(O)R^{c}, (C₀-C₃alkylene)C(O)OR^{c}, (C₀-C₃alkylene)SR^{c}, (C₀-C₃alkylene)C(O)SR^{c}, (C₀-C₃alkylene)SC(O)R^{c}, (C₀-C₃alkylene)C(O)NR^{c}R^{d}, (C₀-C₃alkylene)NC(O)NR^{c}R^{d}, (C₀-C₃alkylene)C(NR^{c})NR^{c}R^{d}, (C₀-C₃alkylene)NR^{c}C(NR^{c})NR^{c}R^{d}, (C₀-C₃alkylene)P(O)OₙR^{c}R^{d}, (C₀-C₃alkylene)S(O)ₘNR^{c}R^{d}, (C₀-C₃alkylene)S(O)ₘOR^{c}, or (C₀-C₃alkylene)BOₚR^{c}R^{d}. In another embodiment, R⁵ is (C₀-C₃alkylene)C₆-C₁₄aryl substituted with one or more substituents selected from cyano, halo, SeH, (C₀-C₃alkylene)NR^{c}R^{d}, (C₀-C₃alkylene)OR^{c}, (C₀-C₃alkylene)OC(O)R^{c}, (C₀-C₃alkylene)C(O)OR^{c}, (C₀-C₃alkylene)SR^{c}, (C₀-C₃alkylene)C(O)SR^{c}, (C₀-C₃alkylene)SC(O)R^{c}, (C₀-C₃alkylene)C(O)NR^{c}R^{d}, (C₀-C₃alkylene)NC(O)NR^{c}R^{d}, (C₀-C₃alkylene)C(NR^{c})NR^{c}R^{d}, (C₀-C₃alkylene)NR^{c}C(NR^{c})NR^{c}R^{d}, (C₀-C₃alkylene)P(O)OₙR^{c}R^{d}, (C₀-C₃alkylene)S(O)ₘNR^{c}R^{d}, (C₀-C₃alkylene)S(O)ₘOR^{c}, or (C₀-C₃alkylene)BOₚR^{c}R^{d}. In another embodiment, R⁵ is (C₀-C₃alkylene)heteroaryl substituted with one or more substituents selected from cyano, halo, SeH, (C₀-C₃alkylene)NR^{c}R^{d}, (C₀-C₃alkylene)OR^{c}, (C₀-C₃alkylene)OC(O)R^{c}, (C₀-C₃alkylene)C(O)OR^{c}, (C₀-C₃alkylene)SR^{c}, (C₀-C₃alkylene)C(O)SR^{c}, (C₀-C₃alkylene)SC(O)R^{c}, (C₀-C₃alkylene)C(O)NR^{c}R^{d}, (C₀-C₃alkylene)NC(O)NR^{c}R^{d}, (C₀-C₃alkylene)C(NR^{c})NR^{c}R^{d}, (C₀-C₃alkylene)NR^{c}C(NR^{c})NR^{c}R^{d}, (C₀-C₃alkylene)P(O)OₙR^{c}R^{d}, (C₀-C₃alkylene)S(O)ₘNR^{c}R^{d}, (C₀-C₃alkylene)S(O)ₘOR^{c}, or (C₀-C₃alkylene)BOₚR^{c}R^{d}.

In another embodiment, R⁶ is H or C₁-C₆ alkyl. In another embodiment, R⁶ is H. In another embodiment, R⁶ is C₁-C₆ alkyl. In another embodiment, R⁶ is C₁-C₆ alkyl substituted with one or more substituents selected from cyano, halo, (C₀-C₃alkylene)NR^{c}R^{d}, or (C₀-C₃alkylene)OR^{c}.

In another embodiment, R⁷ is H or C₁-C₆ alkyl. In another embodiment, R⁷ is H. In another embodiment, R⁷ is C₁-C₆ alkyl. In another embodiment, R⁷ is C₁-C₆ alkyl substituted with one or more substituents selected from cyano, halo, (C₀-C₃alkylene)NR^{c}R^{d}, or (C₀-C₃alkylene)OR^{c}.

In another embodiment, R⁸ is H or C₁-C₆ alkyl. In another embodiment, R⁸ is H. In another embodiment, R⁸ is C₁-C₆ alkyl. In another embodiment, R⁸ is C₁-C₆ alkyl substituted with one or more substituents selected from cyano, halo, (C₀-C₃alkylene)NR^{c}R^{d}, or (C₀-C₃alkylene)OR^{c}.

Yet in another embodiment, R⁴ and R⁶, together with the atoms to which they are attached, may form a 5-membered ring. In another embodiment, R⁴ and R⁶, together with the atoms to which they are attached, may form a 5-membered ring substituted with one or more substituents selected from cyano, halo, (C₀-C₃alkylene)NR^{c}R^{d}, or (C₀-C₃alkylene)OR^{c}. In another embodiment, R⁴ and R⁶, together with the atoms to which they are attached, may form a 6-membered ring. In another embodiment, R⁴ and R⁶, together with the atoms to which they are attached, may form a 6-membered ring substituted with one or more substituents selected from cyano, halo, (C₀-C₃alkylene)NR^{c}R^{d}, or (C₀-C₃alkylene)OR^{c}.

Yet in another embodiment, R⁷ and R⁸, together with the atoms to which they are attached, may form a 5-membered ring. In another embodiment, R⁷ and R⁸, together with the atoms to which they are attached, may form a 5-membered ring substituted with one or more substituents selected from cyano, halo, (C₀-C₃alkylene)NR^{c}R^{d}, or (C₀-C₃alkylene)OR^{c}. In another embodiment, R⁷ and R⁸, together with the atoms to which they are attached, may form a 6-membered ring. In another embodiment, R⁷ and R⁸, together with the atoms to which they are attached, may form a 6-membered ring substituted with one or more substituents selected from cyano, halo, (C₀-C₃alkylene)NR^{c}R^{d}, or (C₀-C₃alkylene)OR^{c}.

In a further embodiment, R^{d} is independently, at each occurrence, H or C₁-C₆alkyl. In another embodiment, R^{d} is H. In another embodiment, R^{d} is C₁-C₆alkyl. In another embodiment, R^{d} is C₁-C₆alkyl one or more substituents selected from (C₀-C₃alkylene)C₃-C₈cycloalkyl, (C₀-C₃alkylene)heterocycloalkyl, (C₀-C₃alkylene)C₆-C₁₄aryl, or (C₀-C₃alkylene)heteroaryl.

In a further embodiment, R^{c} is independently, at each occurrence, H or C₁-C₆alkyl. In another embodiment, R^{c} is H. In another embodiment, R^{c} is C₁-C₆alkyl. In another embodiment, R^{c} is C₁-C₆alkyl one or more substituents selected from (C₀-C₃alkylene)C₃-C₈cycloalkyl, (C₀-C₃alkylene)heterocycloalkyl, (C₀-C₃alkylene)C₆-C₁₄aryl, or (C₀-C₃alkylene)heteroaryl.

In another embodiment, k at each occurrence is 1, 2, 3, 4, 5, 6, 7, or 8. In another embodiment, k is 1. In another embodiment, k is 2. In another embodiment, k is 3. In another embodiment, k is 4. In another embodiment, k is 5. In another embodiment, k is 6. In another embodiment, k is 7. In another embodiment, k is 8.

In one embodiment, m is 0, 1, or 2. In another embodiment, m is 0. In another embodiment, m is 1. In another embodiment, m is 2.

In one embodiment, n is 0, 1, or 2. In another embodiment, n is 0. In another embodiment, n is 1. In another embodiment, n is 2.

In one embodiment, p is 0, 1, or 2. In another embodiment, p is 0. In another embodiment, p is 1. In another embodiment, p is 2.

In some embodiments, the salt of Formula I has the structure of Formula (Ia):

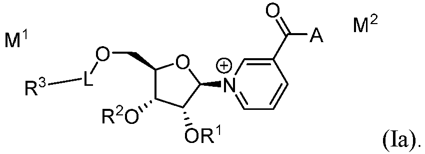

In some embodiments, the salt of Formula I has the structure of Formula Ib:

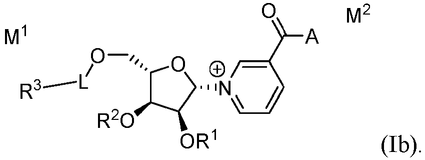

In some embodiments, the salt of Formula I has the structure of Formula Ic:

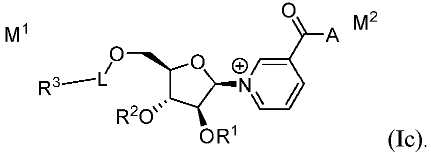

In some embodiments, the salt of Formula I has the structure of Formula Id:

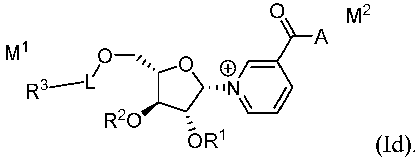

In some embodiments, the salt of Formula I has the structure of Formula Ie:

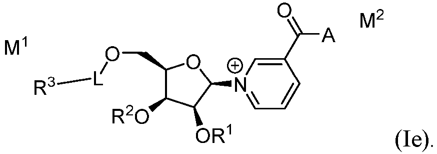

In some embodiments, the salt of Formula I has the structure of Formula If:

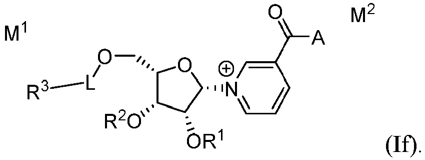

In some embodiments, the salt of Formula I has the structure of Formula Ig:

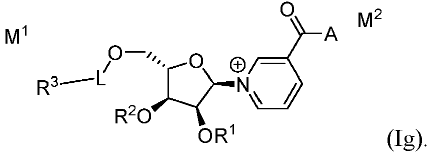

In some embodiments, the salt of Formula I has the structure of Formula Ik:

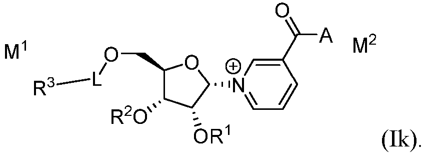

In some embodiments, the salt of Formula I has the structure of Formula Il:

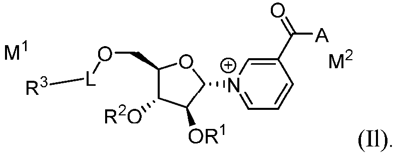

In some embodiments, the salt of Formula I has the structure of Formula Im:

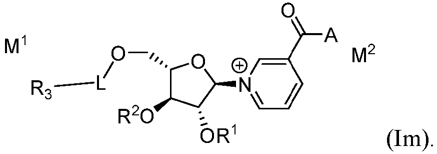

In some embodiments, the salt of Formula I has the structure of Formula In:

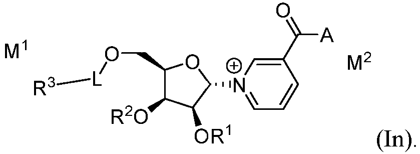

In some embodiments, the salt of Formula I has the structure of Formula Io:

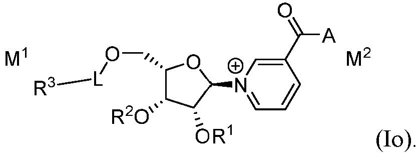

In some embodiments, the salt of Formula I has the structure of Formula Ip:

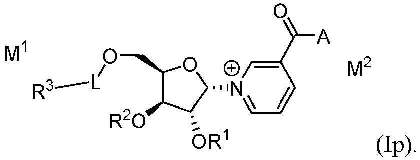

In some embodiments, the salt of Formula I has the structure of Formula Iq;

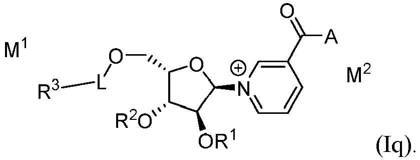

In some embodiments, the salt of Formula I has the structure of Formula Ir:

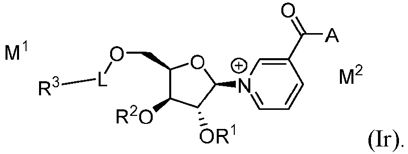

In some embodiments, the salt of Formula I has the structure of Formula Is:

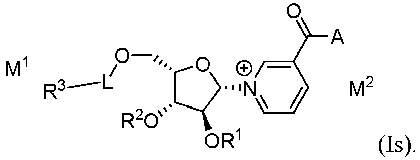

In another embodiment, a suitable salt includes, without limitation: 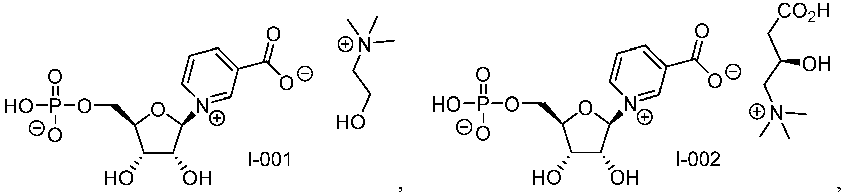 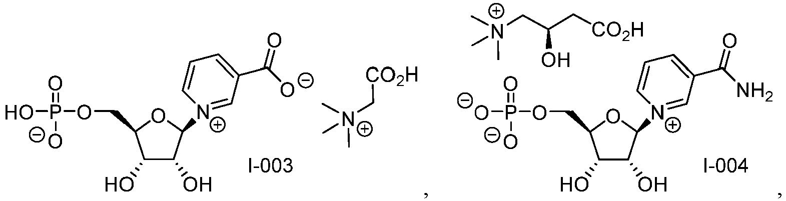 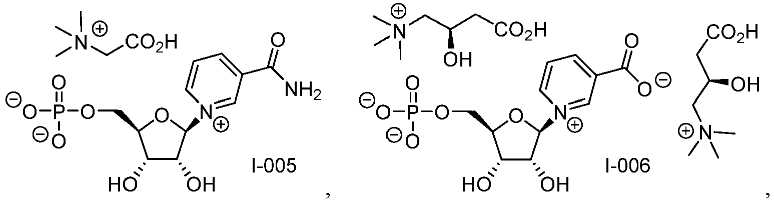 or

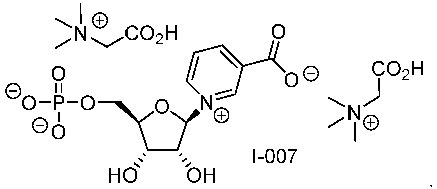

### Method for Preparation of the salts

The salts of the present application may be made by a variety of methods, including standard chemistry. Suitable synthetic routes are depicted in the Schemes given below.

The salts of Formula (I) may be prepared by methods known in the art of organic synthesis as set forth in part by the following synthetic schemes. In the schemes described below, it is well understood that protecting groups for sensitive or reactive groups are employed where necessary in accordance with general principles or chemistry. Protecting groups are manipulated according to standard methods of organic synthesis (T. W. Greene and P. G. M. Wuts, "Protective Groups in Organic Synthesis", Third edition, Wiley, New York 1999). These groups are removed at a convenient stage of the salt synthesis using methods that are readily apparent to those skilled in the art. The selection processes, as well as the reaction conditions and order of their execution, shall be consistent with the preparation of salts of Formula (I).

Those skilled in the art will recognize if a stereocenter exists in the salts of Formula (I). Accordingly, the present application includes both possible stereoisomers (unless specified in the synthesis) and includes not only racemic salts but the individual enantiomers and/or diastereomers as well. When a compound or salt is desired as a single enantiomer or diastereomer, it may be obtained by stereospecific synthesis or by resolution of the final product or any convenient intermediate. Resolution of the final product, an intermediate, or a starting material may be affected by any suitable method known in the art. See, for example, "Stereochemistry of Organic Compounds" by E. L. Eliel, S. H. Wilen, and L. N. Mander (Wiley-Interscience, 1994).

The salts and compounds described herein may be made from commercially available starting materials or synthesized using known organic, inorganic, and/or enzymatic processes.

The salts of the present application can be prepared in a number of ways well known to those skilled in the art of organic synthesis. By way of example, salts of the present application can be synthesized using the methods described below, together with synthetic methods known in the art of synthetic organic chemistry, or variations thereon as appreciated by those skilled in the art. These methods include but are not limited to those methods described below. Salts of the present application can be synthesized by following the steps outlined in General Schemes 1 and 2 which comprise different sequences of assembling various intermediates. Starting materials are either commercially available or made by known procedures in the reported literature or as illustrated.

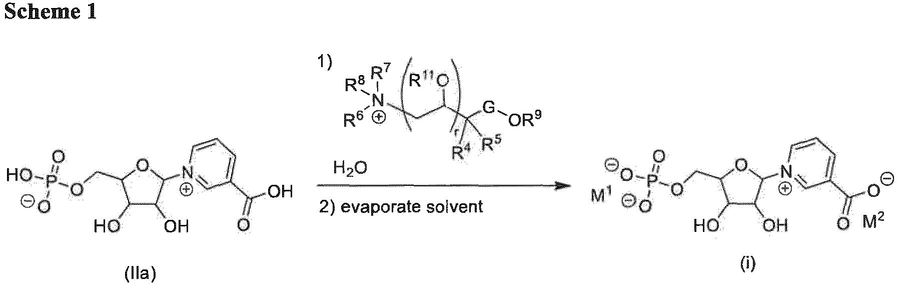

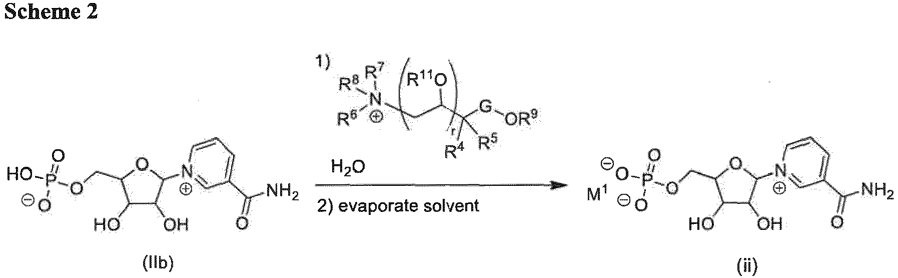

A mixture of enantiomers, diastereomers, cis/trans isomers resulting from the process described above can be separated into their single components by chiral salt technique, chromatography using normal phase, reverse phase or chiral column, depending on the nature of the separation.

It should be understood that in the description and formula shown above, the groups R in the schemes represent R⁴ and R⁵ and other variables are as defined above, except where otherwise indicated. Furthermore, for synthetic purposes, the salts of General Schemes 1 and 2 are mere representative with elected radicals to illustrate the general synthetic methodology of the salts of Formula (I) as defined herein.

It is also understood that the salts disclosed herein possess a neutral electrical charge and that the structure of Formula I is only representative of genus which, if necessary, may be balanced with counterion to allow the salt to present a neutral electrical charge. Such counterions may include, without limitation, bromine, chlorine, and triflates. In one embodiment, the salt of this invention can be generated *in situ* without the need to isolate from solution. In some embodiments, the salts disclosed herein can be discrete 1:1 or 1:2 salts. In some embodiments, the salts can also exist in other ratios, *e.g.,* 1:1.5, 1:5, and 1:10.

### Methods of Using the Disclosed Salts

Salts of the present disclosure are useful in methods of treating or preventing a disease or disorder associated with aging, cellular degradation, and/or cellular restoration. Non limiting examples of such diseases and disorders include infertility, age related infertility, age-related loss of eye function, reduction in bone density, obesity and insulin insensitivity. In one embodiment, the salts of Formula (I) are useful in the treatment of age related infertility. In another embodiment the salts of Formula (I) are useful in the treatment of fertility.

Salts of the present disclosure are useful in methods of treating or preventing an age-related disease or disorder. The method comprises administering to a subject in need thereof a therapeutically effective amount of a pharmaceutical composition of the salt of Formula I.

Yet another aspect of the present disclosure relates to the method of improving oocyte or blastocyst quality and maturation. The method comprises contacting the oocyte or blastocyst for an effective period of time with IVF media comprising a salt of Formula (I).

In another aspect, the present disclosure provides media containing a salt of Formula (I). The salts of Formula (I) have shown surprising and unexpected prolonged stability in solution and thus are useful in media for exposing eggs, oocytes and/or blastocysts for periods of time necessary for enhancing NAD⁺ production prior to implantation into a subject suffering from infertility or age-related infertility. In some embodiments, media comprising a salt of Formula (I) is provided. In some embodiments the media comprises the various reagents and factors necessary for the egg, oocyte or blastocyst depending on which stage of maturation and development the egg, oocyte or blastocyst is in. For example, the media can contain any of the agents or factors useful in IVF media listed in Table 1 below:

**Table 1**

| **CULTURE MEDIA COMPONENTS** |
|---|
| **Inorganic salts** |
| **Energy substrates** (glucose, pyruvate and lactate) |
| **Essential amino acids** (arginine, cysteine, glutamine, histidine, isoleucine, leucine, lysine, methionine, phenylalanine, threonine, tryptophan, tyrosine and valine) |
| **Nonessential amino acids** (alanine, asparagine, aspartate, glycine, glutamate, proline and serine) |
| **Chelators** |
| **pH indicators** |
| **Antibiotic agents** (such as combination of penicillin and streptomycin) |
| **Serum albumin** |
| **Vitamins** |
| **Growth factors** (insulin or GM-CSF, among others) |

Also provided is a cell culture medium for in vitro fertilization comprising: one or more salts of Formula (I) and culturing agents.

In one embodiment, the culturing agent is an inorganic salt, an energy substrate, an amino acid, a chelator, a pH indicator, an antibiotic, a serum, a vitamin, a growth factor, or any combination thereof. In one embodiment, the inorganic salt is calcium chloride, magnesium chloride, magnesium sulfate, potassium chloride, sodium bicarbonate, sodium chloride, monosodium phosphate, disodium phosphate, or any combination thereof.

In one embodiment, the energy substrate is glucose, pyruvate, lactate, pyruvate, or any combination thereof.

In one embodiment, the amino acid is an essential amino acid. In one embodiment, the essential amino acid is arginine, cysteine, glutamine, glycine, histidine, isoleucine, leucine, lysine, methionine, phenylalanine, proline, threonine, tryptophan, tyrosine, valine, or any combination thereof.

In one embodiment, the amino acid is a non-essential amino acid.

In one embodiment, the non-essential amino acid is alanine, asparagine, aspartate, glutamate, proline, serine, or any combination thereof.

In one embodiment, the chelator is clathro chelate, acetyl acetone, amino polycarboxylic acid, ATMP, BAPTA, BDTH2, citric acid, cryptand, deferasirox, 2,3-dihydrobenzoic acid, 2,3-dimercapto-1-propane sulfonic acid, dimercapto succinic acid, DOTA, DTPMP, EDDHA, EDDS, EDTMP, etidronic acid, fura-2, gluconic acid, homocitric acid, iminodiacetic acid, Indo-1, nitrile triacetic acid, pentetic acid (DTPA), phosphonate, phytochelati, poly aspartic acid, sodium poly aspartate, trisodium citrate, transferrin, EDTA, EGTA, or any combination thereof.

In one embodiment, the pH indicator is phenol red, bromothymol blue, alizarin red, 9-amino acridine, or any combination thereof.

In one embodiment, the antibiotic is actinomycin D, ampicillin, carbenicillin, cefotaxime, fosmidomycin, gentamicin, kanamycin, neomycin, penicillin, polymyxin B, streptomycin, or any combination thereof.

In one embodiment, the serum is human serum albumin, bovine serum albumin, fetal bovine serum, synthetic serum, or any combination thereof.

In one embodiment, the vitamin is ascorbic acid, biotin, menadione sodium bisulfite, mitomycin C, pyridoxamine dihydrochloride, retinyl acetate, (-)-riboflavin, (+)-sodium L-ascorbate, (+)-α-tocopherol, vitamin B₁₂, thiamine hydrochloride, i-inositol, pyridoxal hydrochloride, nicotinamide, folic acid, D-calcium pantothenate, choline chloride, or any combination thereof.

In one embodiment, the growth factor is adrenomedullin, angiopoietin, bone morphogenetic proteins, macrophage colony-stimulating factor (M-CSF), granulocyte colony-stimulating factor (G-CSF), granulocyte macrophage colony-stimulating factor (GM-CSF), epidermal growth factor, ephrins, erythropoietin, gibroblast growth factor, growth differentiation factor-9, hepatocyte growth factor, insulin, insulin-like growth factors, interleukins, keratinocyte growth factor, migration-stimulating factor, macrophage-stimulating protein, myostatin, neurotrophins, t-cell growth factor, thrombopoietin, transforming growth factor, tumor necrosis factor-alpha, vascular endothelial growth factor, or any combination thereof.

In one embodiment, the cell culture medium further comprises an oocyte, zygote, blastocyst, or any combination thereof.

Also, provided are kits for IVF media comprising various agents, and factors necessary for oocyte or blastocyst maturation including one or more salts of Formula (I). These agents and cofactors can be dissolved in solution to create the IVF media shortly before use in exposing an oocyte or blastocyst prior to implanting into a patient in need of treatment for infertility or age-related infertility.

Another aspect of the present invention is a pharmaceutical composition comprising the salt of Formula I and a pharmaceutically acceptable carrier.

Another aspect of the present invention is a pharmaceutical composition comprising the salt of Formula I and a pharmaceutically acceptable carrier comprising therapeutically effective amounts of one or more additional therapeutic agents.

In some embodiments, administration of a salt of Formula (I) or a pharmaceutical composition comprising a salt of the present invention and a pharmaceutically acceptable carrier induces a change in the cell cycle or cell viability.

In some embodiments, administration of a salt of Formula (I) or a pharmaceutical composition comprising a salt of the present invention and a pharmaceutically acceptable carrier induces a prophylactic change in the disorder or disease associated with aging.

Administration of the disclosed salts can be accomplished via any mode of administration for therapeutic agents. These modes include systemic or local administration such as oral, nasal, parenteral, transdermal, subcutaneous, vaginal, buccal, rectal or topical administration modes.

Depending on the intended mode of administration, the disclosed compositions can be in solid, semi-solid or liquid dosage form, such as, for example, injectables, tablets, suppositories, pills, time-release capsules, elixirs, tinctures, emulsions, syrups, powders, liquids, suspensions, or the like, sometimes in unit dosages and consistent with conventional pharmaceutical practices. Likewise, they can also be administered in intravenous (both bolus and infusion), intraperitoneal, subcutaneous or intramuscular form, and all using forms well known to those skilled in the pharmaceutical arts.

Pharmaceutical compositions can be prepared according to conventional mixing, granulating or coating methods, respectively, and the present pharmaceutical compositions can contain from about 0.1% to about 99%, from about 5% to about 90%, or from about 1% to about 20% of the disclosed salt by weight or volume.

In one embodiment, the present invention relates to a method of preparing a pharmaceutical composition of the present invention by mixing at least one pharmaceutically acceptable salt of the present invention, and, optionally, one or more pharmaceutically acceptable carriers, additives, or excipients.

In another embodiment, the present invention relates to a method of preparing a pharmaceutical composition of the present invention by mixing at least one pharmaceutically acceptable salt of the present invention and one or more additional therapeutic agents.

Effective dosage amounts of the salts of Formula (I), when used in the described methods, range from about 0.5 mg to about 5000 mg of the disclosed salt as needed to treat the condition. Compositions for in vivo or in vitro use can contain about 0.5, 5, 20, 50, 75, 100, 150, 250, 500, 750, 1000, 1250, 2500, 3500, or 5000 mg of the disclosed salt or, in a range of from one amount to another amount in the list of doses. In one embodiment, the compositions are in the form of a tablet that can be scored.

The dosage regimen utilizing the disclosed salt is selected in accordance with a variety of factors including type, species, age, weight, sex and medical condition of the patient; the severity of the condition to be treated; the route of administration; the renal or hepatic function of the patient; and the particular disclosed salt employed. A physician or veterinarian of ordinary skill in the art can readily determine and prescribe the effective amount of the drug required to prevent, counter or arrest the progress of the condition.

Illustrative pharmaceutical compositions are tablets and gelatin capsules comprising a salt of the Invention and a pharmaceutically acceptable carrier, such as a) a diluent, e.g., purified water, triglyceride oils, such as hydrogenated or partially hydrogenated vegetable oil, or mixtures thereof, corn oil, olive oil, sunflower oil, safflower oil, fish oils, such as EPA or DHA, or their esters or triglycerides or mixtures thereof, omega-3 fatty acids or derivatives thereof, lactose, dextrose, sucrose, mannitol, sorbitol, cellulose, sodium, saccharin, glucose and/or glycine; b) a lubricant, e.g., silica, talcum, stearic acid, its magnesium or calcium salt, sodium oleate, sodium stearate, magnesium stearate, sodium benzoate, sodium acetate, sodium chloride and/or polyethylene glycol; for tablets also; c) a binder, e.g., magnesium aluminum silicate, starch paste, gelatin, tragacanth, methylcellulose, sodium carboxymethylcellulose, magnesium carbonate, natural sugars such as glucose or beta-lactose, corn sweeteners, natural and synthetic gums such as acacia, tragacanth or sodium alginate, waxes and/or polyvinylpyrrolidone, if desired; d) a disintegrant, e.g., starches, agar, methyl cellulose, bentonite, xanthan gum, algic acid or its sodium salt, or effervescent mixtures; e) absorbent, colorant, flavorant and sweetener; f) an emulsifier or dispersing agent, such as Tween 80, Labrasol, HPMC, DOSS, caproyl 909, labrafac, labrafil, peceol, transcutol, capmul MCM, capmul PG-12, captex 355, gelucire, vitamin E TGPS or other acceptable emulsifier; and/or g) an agent that enhances absorption of the salt such as cyclodextrin, hydroxypropyl-cyclodextrin, PEG400, and/or PEG200.

For preparing pharmaceutical compositions from the salts of Formula (I), inert, pharmaceutically acceptable carriers can be either solid or liquid. Solid form preparations include powders, tablets, dispersible granules, capsules, cachets and suppositories. The powders and tablets may be comprised of from about 5 to about 95 percent active ingredient. Suitable solid carriers are known in the art, e.g., magnesium carbonate, magnesium stearate, talc, sugar or lactose. Tablets, powders, cachets and capsules can be used as solid dosage forms suitable for oral administration. Examples of pharmaceutically acceptable carriers and methods of manufacture for various compositions may be found in A. Gennaro (ed.), Remington's Pharmaceutical Sciences, 18th Edition, (1990), Mack Publishing Co., Easton, Pa.

Liquid form preparations include solutions, suspensions and emulsions. For example, water or water-propylene glycol solutions for parenteral injection or addition of sweeteners and opacifiers for oral solutions, suspensions and emulsions. Liquid form preparations may also include solutions for intranasal administration.

Liquid, particularly injectable, compositions can, for example, be prepared by dissolution, dispersion, etc. For example, the disclosed salt is dissolved in or mixed with a pharmaceutically acceptable solvent such as, for example, water, saline, aqueous dextrose, glycerol, ethanol, and the like, to thereby form an injectable isotonic solution or suspension. Proteins such as albumin, chylomicron particles, or serum proteins can be used to solubilize the disclosed compounds.

Parental injectable administration is generally used for subcutaneous, intramuscular or intravenous injections and infusions. Injectables can be prepared in conventional forms, either as liquid solutions or suspensions or solid forms suitable for dissolving in liquid prior to injection.

Aerosol preparations suitable for inhalation may include solutions and solids in powder form, which may be in combination with a pharmaceutically acceptable carrier, such as an inert compressed gas, e.g., nitrogen.

Also included are solid form preparations that are intended to be converted, shortly before use, to liquid form preparations for either oral or parenteral administration. Such liquid forms include solutions, suspensions and emulsions.

### Examples

The disclosure is further illustrated by the following examples and synthesis schemes, which are not to be construed as limiting this disclosure in scope to the specific procedures herein described. It is to be understood that the examples are provided to illustrate certain embodiments and that no limitation to the scope of the disclosure is intended thereby.

The following salts disclosed herein were prepared using the general synthetic methodology including without limitation reagents such as valine, leucine, alanine, isoleucine, methionine, phenylalanine, tryptophan, and tyrosine. Suitable solvents such as methanol, ethanol, water, acetic acid, ethylene glycol, isopropanol were also used.
*Abbreviations used in the following examples and elsewhere herein are:*
- AcOH: acetic acid
- anh.: anhydrous
- atm: atmosphere
- aq.: aqueous
- br: broad
- Boc: *tert*-butyloxycarbonyl
- brine: saturated aqueous sodium chloride
- n-BuLi: *n*-butyllithium
- n-BuOH: *n*-butanol
- Calc'd: calculated
- CDCl₃: deuterated chloroform
- CDI: carbonyldiimidazole
- Chloroform-d: deuterated chloroform
- d: doublet
- dd: doublet of doublets
- dt: doublet of triplets
- D₂O: deuterated water (deuterium oxide)
- DCE: dichloroethane
- DCM: dichloromethane
- DIAD: diisopropyl azodicarboxylate
- DIPEA: *N*,*N*-diisopropylethylamine
- DMAc: *N*,*N*-dimethyl acetamide
- DMAP: *N*,*N*-dimethylpyridin-4-amine
- DME: 1,2-dimethoxyethane
- DMEDA: *N*,*N*'-dimethylethylenediamine
- DMF: *N*,*N*-dimethylformamide
- DMSO: dimethyl sulfoxide
- DMSO-*d₆*: deuterated dimethyl sulfoxide
- EDA: ethylenediamine
- EDC: 1-ethyl-3-(3-dimethylaminopropyl)carbodiimide
- Et₂O: diethyl ether
- EtOAc: ethyl acetate
- EtOH: ethanol
- ESI: electrospray ionization
- g: gram
- h: hour(s)
- H: hydrogen
- ¹H NMR: nuclear magnetic resonance (proton nucleus)
- HATU: [bis(dimethylamino)methylene]-1H-1,2,3-triazolo[4,5-b]pyridinium 3-oxide hexafluorophosphate
- HBTU: 3-[bis(dimethylamino)methylene]-3H-benzotriazol-1-oxide hexafluorophosphate
- HOBt: hydroxybenzotriazole
- HPLC: high pressure (or performance) liquid chromatography
- Hz: hertz
- J: coupling constant
- KHCO₃: potassium bicarbonate
- KHMDS: potassium hexamethyldisilazide
- KOAc: potassium acetate
- LCMS: liquid chromatography mass spectrometry
- LHMDS: lihtium hexamethyldisilazide
- [#] M: molar concentration
- m: multiplet
- [M+H]⁺: molecular ion plus hydrogen
- [M-tBu+H]⁺: molecular ion minus *tert*-butyl plus hydrogen
- mCPBA: *meta*-chloroperoxybenzoic acid
- Me₂NH: dimethylamine
- Me₄NBr: tetramethylammonium bromide
- MeCN: acetonitrile
- MeNH₂: methylamine
- MeOH: methanol
- Methanol-*d*₄: deuterated methanol
- 2-MeTHF: 2-methyl tetrahydrofuran
- mg: milligram
- MHz: megahertz
- min: min
- mmol: millimole
- mL: milliliter
- MS: mass spectrometry
- MS ES: mass spectrometry electrospray
- Ms₂O: methanesulfonic anhydride
- MTBE: methyl *tert*-butyl ether
- MW: microwave
- *m*/*z*: mass-to-charge ratio
- µL: microliter
- N₂: nitrogen
- NaHCO₃: sodium bicarbonate
- NaMN: nicotinic acid mononucleotide
- NIS: *N*-iodosuccinimide
- NMP: *N*-methyl-2-pyrrolidone
- NMR: nuclear magnetic resonance
- PEPPSI-*i*Pr: [1,3-bis(2,6-diisopropylphenyl)imidazol-2-ylidene](3-chloropyridyl)palladium(II) dichloride
- PdCl₂(Amphos): bis(di-*tert*-butyl(4-dimethylaminophenyl)phosphine) dichloropalladium(II)
- Pd₂(dba)₃: tris(dibenzylideneacetone)dipalladium(0)
- Pd(OAc)₂: palladium(II) acetate
- PdCl₂(dppf): [1,1'-bis(diphenylphosphino)ferrocene]dichloropalladium(II)
- PdCl₂(MeCN)₂: bis(acetonitrile)dichloropalladium(II)
- PdCl₂(PPh₃)₂: bis(triphenylphosphinepalladium(II) dichloride
- Pd(P(Cy)₃)₂Cl₂: dichlorobis(tricyclohexylphosphine)palladium(II)
- Pd(PPh₃)₄: tetrakis(triphenylphosphine)palladium(0)
- Pd(*t*-Bu₃P)₂: bis(tri-*tert*-butylphosphine)palladium(0)
- pH: potential of hydrogen
- PMB: 4-methoxybenzyl
- PMBCl: 4-methoxybenzyl chloride
- ppm: parts per million
- prep: preparative
- py: pyridine
- q: quartet
- qd: quartet of doublets
- quant.: quantitative
- quin.: quintuplet
- quind: quintuplet of doublets
- RBF: round-bottom flask
- Rt: retention time
- rt: room temperature
- s: singlet
- sat.: saturated
- sat. aq.: saturated aqueous
- SFMCl: 2-(trimethylsilyl)ethoxymethyl chloride
- t: triplet
- *t*-BuLi: *tert*-butyllithium
- td: triplet of doublets
- TMS: trimethylsilyl
- TMSCl: trimethylsilyl chloride
- tt: triplet of triplets
- T3P: polyphosphonic anhydride
- TBAB: tetrabutylammonium bromide
- TEA: triethylamine
- TFA: trifluoroacetic acid
- TFAA: trifluoroacetic anhydride
- THF: tetrahydrofuran
- TLC: thin layer chromatography
- TPPO: triphenylphosphine oxide
- XantPhos: 4,5-bis(diphenylphosphino)-9,9-dimethylxanthene
- XPhos: 2-dicyclohexylphosphino-2',4',6'-triisopropylbiphenyl

A 50 mL 3 N RBF fitted with a water condenser, rubber septum and internal thermometer was charged with NAMN(0.200 g, 5.97 mmol, 1 eq) and 15 ml of distilled deionized water and mixed to form a solution. This solution was cooled using an ice/water bath to bring the internal temperature below 5 °C. To this solution was then added drop wise 5.67 ml of 0.1M Choline hydroxide solution slowly via syringe so as to prevent the temperature from increasing. The resulting solution was stirred for 10 minutes. After this addition the pH was 4.2-4.8.The flask was then removed and the solution transferred to a 100 ml 1N RBF. The solution was then frozen using liquid nitrogen while swirling to make sure the frozen solution coated the whole of the inside of the RBF. While frozen the flask was attached to the freeze dryer and allowed to evaporate overnight. Once dried the product is rendered as a yellow solid.
**Yield:** 253 mg (97 %)
Analytical data. ¹H-NMR (400 MHz, D₂O) δ = 9.32 (s, 1H), 9.25 (d, 1H), 8.96 (d, 1H), 8.24 (t, 1H), 6.21 (d, 1H), 4.64 (p, 1H), 4.575 (t, 1H), 4.48 (m, 1H),4.32 (m, 1H), 4.18 (dq, 1H), 4.08 (m, 2H), 3.53 (m, 2H), 3.22 (s, 9H) ppm.

### Example 2. Synthesis of (R)-3-Carboxy-2-hydroxy-N,N,N-trimethylpropan-1-aminium 1-((2R,3R,4S,5R)-5-(((hydrogenphosphonato)oxy)methyl)-3,4-dihydroxytetrahydrofuran-2-yl)pyridin-1-ium-3-carboxylate

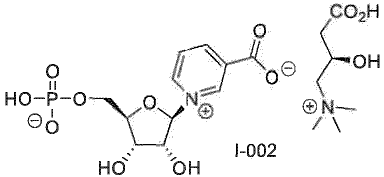

A 100 mL 3 N RBF fitted with a water condenser and internal thermometer was charged with NaMN (0.300 g, 0.894mmo1, 1 eq) and 30 ml of distilled deionized water and mixed to form a solution, a faint suspension is also acceptable. This solution was cooled using an ice/water bath to bring the internal temperature below 10 °C. To this solution was then added dropwise 8.77 ml of 0.1M L- Carnitine solution (0.98 eq) in distilled deionized water slowly so as to prevent the temperature from increasing, all the suspension goes into solution. The resulting solution was stirred for 10 minutes. After this addition the pH was ~ 3.2.The flask was then removed and the colorless solution decanted into a 100 ml 1 N RBF and frozen using liquid nitrogen. While the flask was frozen it was connected to the freeze dryer. Once dried the product is rendered as a colorless to faint yellow solid.
**Yield** :0.3212 g (72% )
**Melting point:** 92 °C (degradation, corrected) 146 °C Outgassing
Analytical data. ¹H-NMR (400 MHz, D₂O) δ = 9.36 (s, 1H), 9.26 (d, 1H), 8.98 (d, 1H), 8.26 (dd, 1H), 6.24 (d, 1H), 4.65 (m, 1H), 4.56 (t, 1H), 4.47 (dd, 1H), 4.33 (dq, 1H), 4.19 (dq, 1H), 3.50 (m, 2H), 3.25 (s, 9H), 2.60-2.70 (m, 2H) ppm.

### Example 3. Synthesis of 1-Carboxy-N,N,N-trimethylmethanaminium 1-((2R,3R,4S,5R)-5-(((hydrogenphosphonato)oxy)methyl)-3,4-dihydroxytetrahydrofuran-2-yl)pyridin-1-ium-3-carboxylate

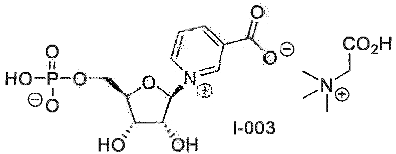

A 50 mL 3 NRBF fitted with a water condenser and internal thermometer was charged with NaMN (0.100 g, 0.298 mmol, 1 eq) and 10 ml of distilled deionized water and mixed to form a solution. This solution was cooled using an ice/water bath to bring the internal temperature below 10 °C. To this solution was then added dropwise 2.92 ml of 0.1M Betaine solution (0.98 eq) in distilled deionized water slowly so as to prevent the temperature from increasing, all the suspension goes into solution. The resulting solution was stirred for 10 minutes. After this addition the pH was ~ 1.8-2.3.The flask was then removed and the colorless solution decanted into a 50 ml 1 N RBF and frozen using liquid nitrogen. While the flask was frozen it was connected to the freeze dryer. Once dried the product is rendered as a colorless to faint yellow solid.
**Yield:** 140.2 mg (quantitative yield)
**Melting point:** 122-133 °C (degradation, corrected)
Analytical data. ¹H-NMR (400 MHz, D₂O) δ = 9.45 (s, 1H), 9.30 (d, 1H), 9.05 (d, 1H), 8.28 (dd, 1H), 6.25 (d, 1H), 4.66 (p, 1H), 4.57 (t, 1H), 4.47 (dd, 1H), 4.33 (dq, 1H), 4.19 (dq, 1H), 4.04 (s, 2H), 3.30 (s, 9H) ppm.

### Example 4. Synthesis of (R)-3-Carboxy-2-hydroxy-N,N,N-trimethylpropan-1-aminium ((2R,3S,4R,5R)-5-(3-carbamoylpyridin-1-ium-1-yl)-3,4-dihydroxytetrahydrofuran-2-yl)methyl phosphate

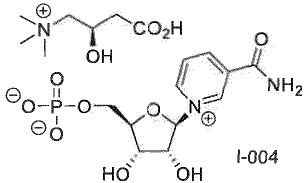

A 50 mL 3 N RBF fitted with a water condenser and internal thermometer was charged with NMN (0.250 g, 0.748mmol, 1 eq) and 25ml of distilled deionized water and mixed to form a solution. This solution was cooled using an ice/water bath to bring the internal temperature below 10 °C. To this solution was then added dropwise 7.33 ml of 0.1M L- Carnitine solution (0.98 eq) in distilled deionized water slowly so as to prevent the temperature from increasing, all the suspension goes into solution. The resulting solution was stirred for 10 minutes. After this addition the pH was ~ 4.4.The flask was then removed and the colorless solution decanted into a 100 ml 1 N RBF and frozen using liquid nitrogen. While the flask was frozen it was connected to the freeze dryer. Once dried the product is rendered as a colorless to faint yellow solid.
**Yield:** 249.4 mg (67 %)
**Melting point:** 68-99 °C (degradation, corrected) outgassing at 136 °C
Analytical data. ¹H-NMR (400 MHz, D₂O) δ = 9.50 (s, 1H), 9.31 (d, 1H), 9.02 (d, 1H), 8.33 (dd, 1H), 6.25 (d, 1H), 4.68 (p, 1H), 4.60 (m, 2H), 4.48 (dd, 1H), 4.33 (dq, 1H), 4.18 (dq, 1H), 3.45 (m, 2 H), 3.25 (s, 9H), 2.48 (m, 2H) ppm.

### Example 5. Synthesis of 1-Carboxy-N,N,N-trimethylmethanaminium ((2R,3S,4R,5R)-5-(3-carbamoylpyridin-1-ium-1-yl)-3,4-dihydroxytetrahydrofuran-2-yl)methyl phosphate

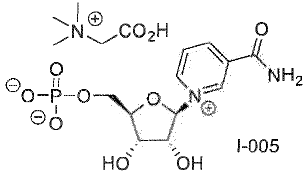

A 50 mL 3 N RBF fitted with a water condenser and internal thermometer was charged with NMN (0.100 g, 0.299 mmol, 1 eq) and 5 ml of distilled deionized water and mixed to form a solution, a faint suspension is also acceptable. This solution was cooled using an ice/water bath to bring the internal temperature below 10 °C. To this solution was then added dropwise 2.93 ml of 0.1M Betaine solution (0.98 eq) in distilled deionized water slowly so as to prevent the temperature from increasing, all the suspension goes into solution. The resulting solution was stirred for 10 minutes. After this addition the pH was ~ 3.8-4.2.The flask was then removed and the colorless solution decanted into a 50 ml 1 N RBF and frozen using liquid nitrogen. While the flask was frozen it was connected to the freeze dryer. Once dried the product is rendered as a colorless to faint yellow solid.
**Yield:** 129.5 mg (97 %)
**Melting point:** 105 °C (melted) 129 °C (degradation, corrected)
Analytical data. ¹H-NMR (400 MHz, D₂O) δ = 9.50 (s, 1H), 9.31 (d, 1H), 9.02 (d, 1H), 8.33 (dd, 1H), 6.25 (d, 1H), 4.68 (m, 1H), 4.60 (t, 1H), 4.47 (dd, 1H), 4.33 (dq, 1H), 4.18 (dq, 1H), 3.92 (s, 2 H), 3.28 (s, 9H) ppm.

### Example 6. Synthesis of (R)-3-Carboxy-2-hydroxy-N,N,N-trimethylpropan-1-aminium 1-((2R,3R,4S,5R)-3,4-dihydroxy-5-((phosphonatooxy)methyl)tetrahydrofuran-2-yl)pyridin-1-ium-3-carboxylate

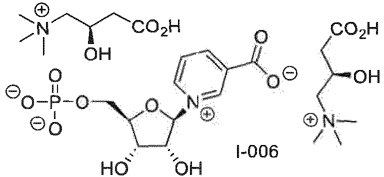

A 100 mL 3 N RBF fitted with a water condenser and internal thermometer was charged with NaMN (0.250 g, 0.298 mmol, 1 eq) and 25ml of distilled deionized water and mixed to form a solution, a faint suspension is also fine. This solution was cooled using an ice/water bath to bring the internal temperature below 10 °C. To this solution was then added dropwise 14.76 ml of 0.1M L-Carnitine solution (1.98 eq) in distilled deionized water slowly so as to prevent the temperature from increasing, all the suspension goes into solution. The resulting solution was stirred for 10 minutes. After this addition the pH was ~ 3.3-3.9. The flask was then removed and the colorless solution decanted into a 100 ml 1 N RBF and frozen using liquid nitrogen. While the flask was frozen it was connected to the freeze dryer. Once dried the product is rendered as a colorless to faint yellow solid.
**Yield:** 238.9 mg (49 % after transfer)
Analytical data. ¹H-NMR (400 MHz, D₂O) δ = 9.35 (s, 1H), 9.25 (d, 1H), 8.95 (d, 1H), 8.25 (dd, 1H), 6.23 (d, 1H), 4.65 (m, 3H), 4.55 (t, 1H), 4.48 (d7, 1H), 4.33 (dq, 1H), 4.18 (dq, 1H), 3.48 (m, 4H), 3.24 (s, 18H), 2.56 (d, 4H) ppm.

### Example 7. Synthesis of 1-Carboxy-N,N,N-trimethylmethanaminium 1-((2R,3R,4S,5R)-3,4-dihydroxy-5-((phosphonatooxy)methyl)tetrahydrofuran-2-yl)pyridin-1-ium-3-carboxylate

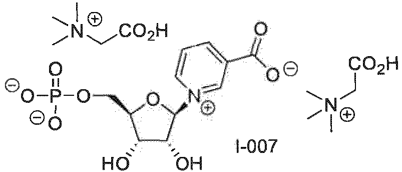

A 50 mL 3 N RBF fitted with a water condenser and internal thermometer was charged with NaMN (0.100 g, 0.298 mmol, 1 eq) and 10 ml of distilled deionized water and mixed to form a solution. This solution was cooled using an ice/water bath to bring the internal temperature below 10 °C. To this solution was then added dropwise 5.85 ml of 0.1M Betaine solution (1.98 eq) in distilled deionized water slowly so as to prevent the temperature from increasing, all the suspension goes into solution. The resulting solution was stirred for 10 minutes. After this addition the pH was ~ 2.8 -2.3. The flask was then removed and the colorless solution decanted into a 50 ml 1 N RBF and frozen using liquid nitrogen. While the flask was frozen it was connected to the freeze dryer. Once dried the product is rendered as a colorless to faint yellow solid.
**Yield:** 167.0 mg (49.5 %)
**Melting point:** 82-102 °C (completely melted) 150 °C (degradation, corrected)
Analytical data. ¹H-NMR (400 MHz, D₂O) δ = 9.42 (s, 1H), 9.30 (d, 1H), 9.02 (d, 1H), 8.28 (dd, 1H), 6.24 (d, 1H), 4.65 (m, 1H), 4.58 (t, 1H), 4.47 (dd, 1H), 4.33 (dq, 1H), 4.19 (dq, 1H), 4.01 (s, 4H), 3.28 (s, 18H) ppm.
**Yield:** 167.0 mg (49.5 % )
**Melting point:** 82-102 °C (completely melted) 150 °C (degradation, corrected)
Analytical data. ¹H-NMR (400 MHz, D₂O) δ = 9.42 (s, 1H), 9.30 (d, 1H), 9.02 (d, 1H), 8.28 (dd, 1H), 6.24 (d, 1H), 4.65 (m, 1H), 4.58 (t, 1H), 4.47 (dd, 1H), 4.33 (dq, 1H), 4.19 (dq, 1H), 4.01 (s, 4H), 3.28 (s, 18H) ppm

### Example 10. NAD Cell Assays

NAD levels were assayed based on the NAD cycling method of Zhu and Rand, PLoS One (2012). COV434 cells were maintained in 6 well plates and treated with the indicated compounds at a concentration of 200 uM for 4 hr. Media was removed, plates were washed in cold PBS and cells were scraped down in NAD extraction buffer containing 10 mM nicotinamide, 50 mM Tris HCl, 0.1% Triton X-100. Cells were homogenized by sonication for 5 seconds, and samples were centrifuged at 7,000 g for 5 min at 4 degrees. Aliquots were taken for later protein assay, and samples were then passed through 10 kDa amicon filters at 14,000 g, 30 min at 4 degrees to remove proteins from the sample. Each sample was measured in technical triplicate, with 25 µL sample added to 100 µL ADH cycling mix (0.2 mg/ml alcohol dehydrogenase enzyme, 2% ethanol, 100 mM Tris pH 8.5). Samples were allowed to cycle for 10 min at room temperature, followed by 50 µL addition of an MTT/PMS solution (0.1 mM phenazine methosulfate, 0.8 mM 3-(4,5-Dimethylthiazol-2-yl)-2,5-diphenyltetrazolium bromide), 100 mM Tris-HCl pH 8.5). Plates were then incubated for 15 min and absorbance was measured at 570 nM. NAD concentrations were extrapolated from a standard curve, and normalized to protein concentrations determined by BCA protein assay.

The results of the assay described above are shown in Table 2 (below). The fold increase is obtained through a direct comparison on a mole per mole between the compared salt and its parent counterpart.

**Table 2**

| Compound ID | Fold Increase of NAD Adjusted by Molecular Weight |
|---|---|
| I-002 | 1.47 |
| I-003 | 1.51 |
| I-005 | 1.35 |
| I-006 | 1.64 |
| I-007 | 1.92 |

## Claims

1. A salt of Formula (I): 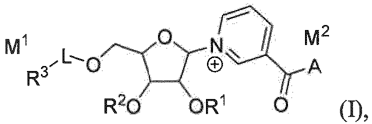
or an enantiomer, stereoisomer, or tautomer thereof,
wherein
A is NR^{a}R^{b} or O⁻;
L is a bond or 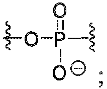 R¹ and R² are independently H, C₁-C₆alkyl, C₁-C₆haloalkyl, -C(O)C₁-C₆haloalkyl, (C₀-C₃alkylene)C(O)C₁-C₆alkyl, -C(O)OR^{a}, -C(O)NR^{a}R^{b},-[CH₂-CH₂-O]ₖ-R^{a}, - C(O)[CH₂-CH₂-O]ₖ-R^{a}, -[CH₂-CH₂-CH₂-O]ₖ-R^{a}, or -C(O)[CH₂-CH₂-CH₂-O]ₖ-R^{a},
or R¹ and R², together with the atom to which they are attached, form a 5-membered heterocyclic ring optionally substituted with one or more substituents selected from C₁-C₆alkyl, C₂-C₆alkenyl, C₂-C₆alkynyl, (C₀-C₃alkylene)C₃-C₈cycloalkyl, (C₀-C₃alkylene)heterocycloalkyl, (C₀-C₃alkylene)C₆-C₁₄aryl, or (C₀-C₃alkylene)heteroaryl;
R³ is a negative charge, H, or C₁-C₆ alkyl;
R^{a} and R^{b} are independently, at each occurrence, H or C₁-C₆alkyl, wherein the alkyl is optionally substituted with one or more substituents selected from (C₀-C₃alkylene)C₃-C₈cycloalkyl, (C₀-C₃alkylene)heterocycloalkyl, (C₀-C₃alkylene)C₆-C₁₄aryl, or (C₀-C₃alkylene)heteroaryl; and
k is an integer from 1 to 8,
M¹ and M² are independently absent or a quaternary cation of Formula (II): 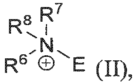 wherein
E is C₁-C₆alkyl optionally substituted with one or more substituents selected from R⁴, R⁵, G-OR⁹, or (C₀-C₃alkylene)OR¹¹;
R⁴ and R⁵ are independently, at each occurrence, H or C₁-C₆alkyl, C₂-C₆alkenyl, C₂-C₆alkynyl, (C₀-C₃alkylene)C₃-C₈cycloalkyl, (C₀-C₃alkylene)heterocycloalkyl, (C₀-C₃alkylene)C₆-C₁₄aryl, or (C₀-C₃alkylene)heteroaryl, wherein the alkyl, alkenyl, alkynyl, alkylene, cycloalkyl, heterocycloalkyl, aryl, or heteroaryl is optionally substituted with one or more substituents selected from cyano, halo, SeH, (C₀-C₃alkylene)NR^{c}R^{d}, (C₀-C₃alkylene)OR^{c}, (C₀-C₃alkylene)OC(O)R^{c}, (C₀-C₃alkylene)C(O)OR^{c}, (C₀-C₃alkylene)SR^{c}, (C₀-C₃alkylene)C(O)SR^{c}, (C₀-C₃alkylene)SC(O)R^{c}, (C₀-C₃alkylene)C(O)NR^{c}R^{d}, (C₀-C₃alkylene)NC(O)NR^{c}R^{d}, (C₀-C₃alkylene)C(NR^{c})NR^{c}R^{d}, (C₀-C₃alkylene)NR^{c}C(NR^{c})NR^{c}R^{d}, (C₀-C₃alkylene)P(O)OₙR^{c}R^{d}, (C₀-C₃alkylene)S(O)ₘNR^{c}R^{d}, (C₀-C₃alkylene)S(O)ₘOR^{c}, or (C₀-C₃alkylene)BOₚR^{c}R^{d};
R⁶, R⁷, and R⁸ are independently H or C₁-C₆alkyl, wherein the alkyl is optionally substituted with one or more substituents selected from cyano, halo, (C₀-C₃alkylene)NR^{c}R^{d}, or (C₀-C₃alkylene)OR^{c},
or R⁴ and R⁶, together with the atoms to which they are attached, form a 5- to 6-membered ring optionally substituted with one or more substituents selected from cyano, halo, (C₀-C₃alkylene)NR^{c}R^{d}, or (C₀-C₃alkylene)OR^{c},
or R⁷ and R⁸, together with the atoms to which they are attached, form a 5- to 6-membered ring optionally substituted with one or more substituents selected from cyano, halo, (C₀-C₃alkylene)NR^{c}R^{d}, or (C₀-C₃alkylene)OR^{c};
R^{c} and R^{d} are independently, at each occurrence, H or C₁-C₆alkyl, wherein the alkyl is optionally substituted with one or more substituents selected from (C₀-C₃alkylene)C₃-C₈cycloalkyl, (C₀-C₃alkylene)heterocycloalkyl, (C₀-C₃alkylene)C₆-C₁₄aryl, or (C₀-C₃alkylene)heteroaryl;
R⁹ is a negative charge, H, or C₁-C₆ alkyl;
R¹¹ is H, C₁-C₆alkyl, or C(O)C₁-C₆alkyl, wherein the C₁-C₆alkyl, or C(O)C₁-C₆alkyl is optionally substituted with one or more substituents selected from (C₀-C₃alkylene)NR^{c}R^{d}, (C₀-C₃alkylene)OR^{c}, (C₀-C₃alkylene)C₃-C₈cycloalkyl, (C₀-C₃alkylene)heterocycloalkyl, (C₀-C₃alkylene)C₆-C₁₄aryl, or (C₀-C₃alkylene)heteroaryl;
G is CH₂ or C(O); and
m, n, and p are independently, at each occurrence, 0, 1, or 2;
provided that
a) at least one of M¹ or M² is a quaternary cation
b) when L is a bond, M¹ is absent, and
c) when A is NR^{a}R^{b}, M² is absent.

2. The salt of claim 1, wherein A is O⁻.

3. The salt of claim 1, wherein A is NR^{a}R^{b}, optionally wherein R^{a} is H or methyl.

4. The salt of any one of claims 1-3, wherein M¹ and M² are independently absent or a quaternary ammonium cation of Formula (IIa): 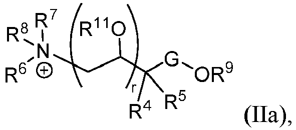 wherein r is 0 or 1.

5. The salt of any one of claims 1-4, wherein R⁵ is H and/or R⁶ is H.

6. The salt of any one of claims 1 to 5, wherein R⁴ is H, or
wherein R⁴ is C₁-C₄alkyl, (C₀-C₃alkylene)C₆-C₁₄aryl, or (C₀-C₃alkylene)heteroaryl, or C₁-C₄alkyl substituted with one or more (C₀-C₃alkylene)SR^{c}.

7. The salt of any one of claims 1 to 6, wherein M¹ is 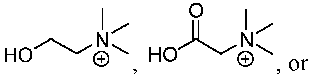 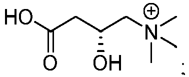 or wherein M¹ and M² are

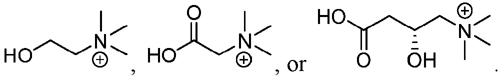

8. The salt of any one of claims 1 to 7, wherein R¹ is H, C(O)C₁-C₃alkyl, or C₁-C₃alkyl;
and/or wherein R² is H, C(O)C₁-C₃alkyl, or C₁-C₃alkyl;
and/or wherein R³ is H or methyl.

9. The salt of claim 1, having the structure 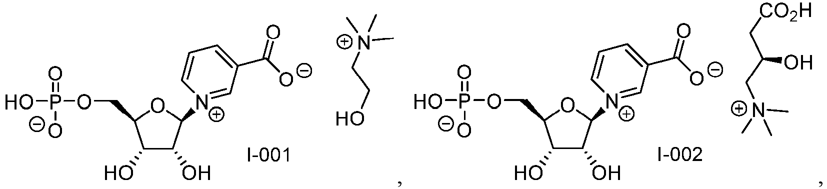 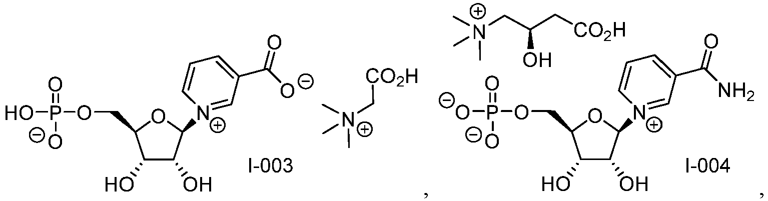 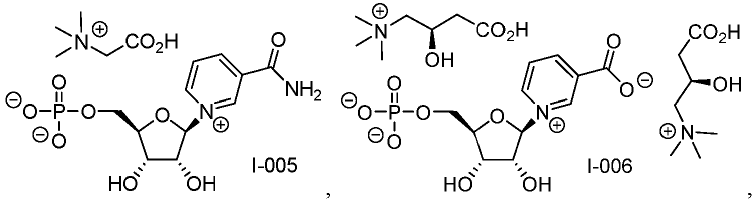 or

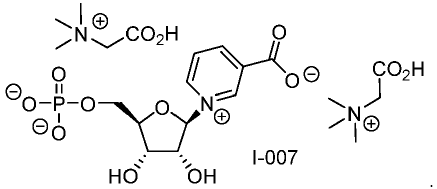

10. A pharmaceutical composition comprising a salt of any one of claims 1 to 9 and a pharmaceutically acceptable carrier.

11. The salt of any one of claims 1-9 or the composition of claim 10, for use in a method of treating or preventing infertility, the method comprising administering to a subject in need thereof an effective amount of the salt or the composition,
optionally wherein the infertility is an age-related infertility.

12. A method of improving oocyte or blastocyst quality comprising contacting the oocyte or blastocyst with an effective amount of a salt of any one of the claims 1 to 9 or the composition of claim 10 prior to implantation into a subject in need of treatment of age-related infertility, optionally wherein the oocyte or blastocyst is cultured in an IVF media containing the salt.

13. A method of improving oocyte or blastocyst maturation comprising contacting the oocyte or blastocyst with an effective amount of a salt of any one of the claims 1 to 9 or the composition of claim 10 prior to implantation into a subject in need of treatment of age-related infertility, optionally wherein the oocyte or blastocyst is cultured in an IVF media containing the salt.

14. A cell culture medium for in vitro fertilization comprising:
a compound selected from any one of claims 1 to 9; and
a culturing agent.

15. The cell culture medium for in vitro fertilization of claim 14, wherein the culturing agent is an inorganic salt, an energy substrate, an amino acid, a chelator, a pH indicator, an antibiotic, a serum, a vitamin, a growth factor, or any combination thereof; optionally wherein:
(a) the inorganic salt is calcium chloride, magnesium chloride, magnesium sulfate, potassium chloride, sodium bicarbonate, sodium chloride, monosodium phosphate, disodium phosphate, or any combination thereof,
(b) the energy substrate is glucose, pyruvate, lactate, pyruvate, or any combination thereof,
(c) the amino acid is an essential amino acid, optionally arginine, cysteine, glutamine, glycine, histidine, isoleucine, leucine, lysine, methionine, phenylalanine, proline, threonine, tryptophan, tyrosine, valine, or any combination thereof,
(d) the amino acid is a non-essential amino acid, optionally alanine, asparagine, aspartate, glutamate, proline, serine, or any combination thereof,
(e) the chelator is clathro chelate, acetyl acetone, amino polycarboxylic acid, ATMP, BAPTA, BDTH2, citric acid, cryptand, deferasirox, 2,3-dihydrobenzoic acid, 2,3-dimercapto-1-propane sulfonic acid, dimercapto succinic acid, DOTA, DTPMP, EDDHA, EDDS, EDTMP, etidronic acid, fura-2, gluconic acid, homocitric acid, iminodiacetic acid, Indo-1, nitrile triacetic acid, pentetic acid (DTPA), phosphonate, phytochelati, poly aspartic acid, sodium poly aspartate, trisodium citrate, transferrin, EDTA, EGTA, or any combination thereof,
(f) the pH indicator is phenol red, bromothymol blue, alizarin red, 9-aminoacridine, or any combination thereof,
(g) the antibiotic is actinomycin D, ampicillin, carbenicillin, cefotaxime, fosmidomycin, gentamicin, kanamycin, neomycin, penicillin, polymyxin B, streptomycin, or any combination thereof, or
(h) the serum is human serum albumin, bovine serum albumin, fetal bovine serum, synthetic serum, or any combination thereof.

## Patentansprüche

1. Salz der Formel (I): 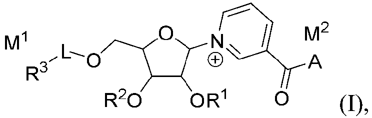
oder ein Enantiomer, Stereoisomer oder Tautomer davon,
wobei
A NR^{a}R^{b} oder O⁻ ist;
L eine Bindung oder 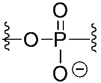 ist;
R¹ und R² unabhängig voneinander H, C₁-C₆ Alkyl, C₁-C₆ Halogenalkyl, - C(O)C₁-C₆ Halogenalkyl, (C₀-C₃ Alkylen)C(O)C₁-C₆ Alkyl, -C(O)OR^{a} , -C(O)NR^{a}R^{b} , -[CH₂ - CH₂-O]ₖ-R^{a}, -C(O)[CH₂-CH₂-O]ₖ-R^{a}, -[CH₂-CH₂-CH₂-O]ₖ-R^{a} oder -C(O)[CH₂-CH₂-CH₂-O]ₖ-R^{a} sind,
oder R¹ und R² zusammen mit dem Atom, an das sie gebunden sind, einen 5-gliedrigen heterocyclischen Ring bilden, der gegebenenfalls mit einem oder mehreren Substituenten substituiert ist, die ausgewählt sind aus C₁-C₆ Alkyl, C₂-C₆ Alkenyl, C₂-C₆ Alkinyl, (C₀-C₃ Alkylen)C₃-C₈ Cycloalkyl, (C₀-C₃ Alkylen)heterocycloalkyl, (C₀-C₃ Alkylen)C₆-C₁₄ Aryl oder (C₀-C₃ Alkylen)heteroaryl;
R³ eine negative Ladung, H oder C₁-C₆ Alkyl ist;
R^{a} und R^{b} unabhängig voneinander bei jedem Vorkommen H oder C₁-C₆ Alkyl sind, wobei das Alkyl gegebenenfalls mit einem oder mehreren Substituenten substituiert ist, die ausgewählt sind aus (C₀-C₃ Alkylen)C₃-C₈ Cycloalkyl, (C₀-C₃ Alkylen)heterocycloalkyl, (C₀-C₃ Alkylen)C₆-C₁₄ Aryl oder (C₀-C₃ Alkylen)heteroaryl; und
k eine ganze Zahl von 1 bis 8 ist,
M¹ und M² unabhängig voneinander nicht vorhanden oder ein quaternäres Kation der Formel (II) sind: wobei 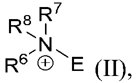
E C₁-C₆ Alkyl ist, das gegebenenfalls mit einem oder mehreren Substituenten substituiert ist, die ausgewählt sind aus
R⁴, R⁵, G-OR⁹ oder (C₀-C₃ Alkylen)OR¹¹;
R⁴ und R⁵ unabhängig voneinander bei jedem Vorkommen H oder C₁-C₆ Alkyl, C₂-C₆ Alkenyl, C₂-C₆ Alkinyl, (C₀-C₃ Alkylen)C₃-C₈ cycloalkyl, (C₀-C₃ alkylen)heterocycloalkyl, (C₀-C₃ alkylen)C₆-C(₁₄₎ Aryl oder (C₀-C₃ Alkylen)heteroaryl sind, wobei das Alkyl, Alkenyl, Alkinyl, Alkylen, Cycloalkyl, Heterocycloalkyl, Aryl oder Heteroaryl gegebenenfalls mit einem oder mehreren Substituenten substituiert ist, die ausgewählt sind aus Cyano, Halogen, SeH, (C₀-C₃ alkylen)NR^{c}R^{d}, (C₀-C₃ Alkylen)OR^{c}, (C₀-C₃ Alkylen)OC(O)R^{c}, (C₀-C₃ Alkylen)C(O)OR^{c}, (C₀-C₃ Alkylen)SR^{c}, (C₀-C₃ Alkylen)C(O)SR^{c}, (C₀-C₃ Alkylen)SC(O)R^{c}, (C₀-C₃ Alkylen)C(O)NR^{c}R^{d}, (C₀-C₃ Alkylen)NC(O)NR^{c}R^{d}, (C₀-C₃ Alkylen)C(NR^{c})NR^{c}R^{d}, (C₀-C₃ Alkylen)NR^{c}C(NR^{c})NR^{c}R^{d}, (C₀-C₃ Alkylen)P(O)OₙR^{c}R^{d}, (C₀-C₃ Alkylen)S(O)ₘNR^{c}R^{d}, (C₀-C₃ Alkylen)S(O)ₘOR^{c} oder (C₀-C₃ Alkylen)BOₚR^{c}R^{d};
R⁶, R⁷ und R⁸ unabhängig voneinander H oder C₁-C₆ Alkyl sind, wobei das Alkyl gegebenenfalls mit einem oder mehreren Substituenten substituiert ist, die ausgewählt sind aus Cyano, Halogen, (C₀-C₃ Alkylen)NR^{c}R^{d} oder (C₀-C₃ Alkylen)OR^{c},
oder R⁴ und R⁶ zusammen mit den Atomen, an die sie gebunden sind, einen 5- bis 6-gliedrigen Ring bilden, der gegebenenfalls mit einem oder mehreren Substituenten substituiert ist, die ausgewählt sind aus Cyano, Halogen, (C₀-C₃ Alkylen)NR^{c}R^{d} oder (C₀-C₃ Alkylen)OR^{c},
oder R⁷ und R⁸ zusammen mit den Atomen, an die sie gebunden sind, einen 5- bis 6-gliedrigen Ring bilden, der gegebenenfalls mit einem oder mehreren Substituenten substituiert ist, die ausgewählt sind aus Cyano, Halogen, (C₀-C₃ Alkylen)NR^{c}R^{d} oder (C₀-C₃ Alkylen)OR^{c};
R^{c} und R^{d} unabhängig voneinander bei jedem Vorkommen H oder C₁-C₆ Alkyl sind, wobei das Alkyl gegebenenfalls mit einem oder mehreren Substituenten substituiert ist, die ausgewählt sind aus (C₀-C₃ Alkylen)C₃-C₈ Cycloalkyl, (C₀-C₃ Alkylen)heterocycloalkyl, (C₀-C₃ Alkylen)C₆-C₁₄ Aryl oder (C₀-C₃ Alkylen)heteroaryl;
R⁹ eine negative Ladung, H oder C₁-C₆ Alkyl ist;
R¹¹ H, C₁-C₆ Alkyl oder C(O)C -C₆ Alkyl ist, wobei das C₁-C₆ Alkyl oder C(O)C₁-C₆ Alkyl gegebenenfalls mit einem oder mehreren Substituenten substituiert ist, die ausgewählt sind aus (C₀-C₃ Alkylen)NR^{c}R^{d}, (C₀-C₃ Alkylen)OR^{c}, (C₀-C₃ Alkylen)C₃-C₈ Cycloalkyl, (C₀-C₃ Alkylen)heterocycloalkyl, (C₀-C₃ Alkylen)C₆-C₁₄ Aryl oder (C₀-C₃ Alkylen)heteroaryl;
G CH₂ oder C(O) ist; und
m, n und p unabhängig voneinander bei jedem Vorkommen 0, 1 oder 2 sind;
mit der Maßgabe, dass
a) mindestens eines von M¹ oder M² ein quaternäres Kation ist
b) wenn L eine Bindung ist, M¹ fehlt, und
c) wenn A NR^{a}R^{b} ist, M² fehlt.

2. Salz nach Anspruch 1, wobei A O⁻ ist.

3. Salz nach Anspruch 1, wobei A NR^{a}R^{b} ist, gegebenenfalls wobei R^{a} H oder Methyl ist.

4. Salz nach einem der Ansprüche 1 bis 3, wobei M¹ und M² unabhängig voneinander nicht vorhanden sind oder ein quaternäres Ammoniumkation der Formel (IIa) sind: 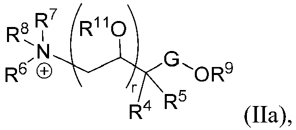 wobei r 0 oder 1 ist.

5. Salz nach einem der Ansprüche 1-4, wobei R⁵ H und/oder R⁶ H ist.

6. Salz nach einem der Ansprüche1 bis 5, wobei R⁴ H ist, oder
wobei R⁴ C₁-C₄ Alkyl, (C₀-C₃ Alkylen)C₆-C₁₄ Aryl oder (C₀-C₃ Alkylen)Heteroaryl oder C₁-C₄ Alkyl, substituiert mit einem oder mehreren (C₀-C₃ Alkylen)SR^{c} ist.

7. Salz nach einem der Ansprüche 1 bis 6, wobei 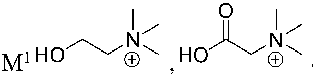 oder 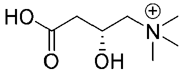 ist; oder wobei M¹ und 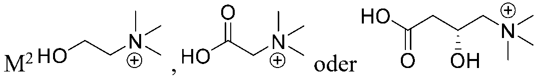 sind.

8. Salz nach einem der Ansprüche 1 bis 7, wobei R¹ H, C(O)C₁-C₃ Alkyl oder C₁-C₃ Alkyl ist;
und/oder wobei R² H, C(O)C₁-C₃ Alkyl oder C₁-C₃ Alkyl ist;
und/oder wobei R³ H oder Methyl ist.

9. Salz nach Anspruch 1 mit der Struktur 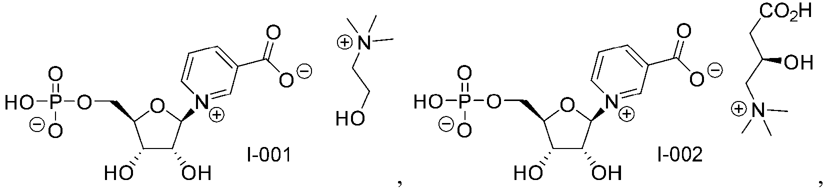 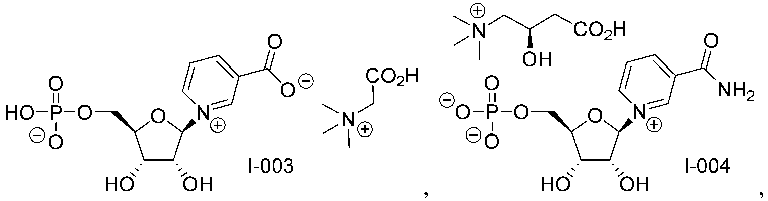 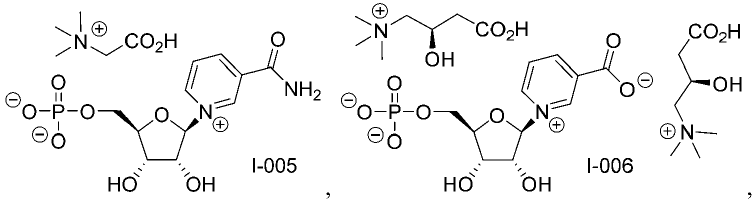 oder

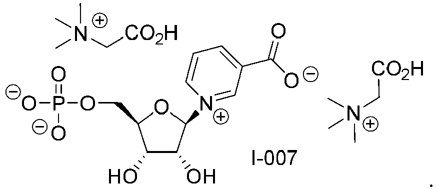

10. Pharmazeutische Zusammensetzung, umfassend ein Salz nach einem der Ansprüche 1 bis 9 und einen pharmazeutisch akzeptablen Träger.

11. Salz nach einem der Ansprüche 1 bis 9 oder die Zusammensetzung nach Anspruch 10 zur Verwendung in einem Verfahren zur Behandlung oder Vorbeugung von Unfruchtbarkeit, wobei das Verfahren die Verabreichung einer wirksamen Menge des Salzes oder der Zusammensetzung an ein Subjekt umfasst, das diese benötigt,
wobei die Unfruchtbarkeit gegebenenfalls eine altersbedingte Unfruchtbarkeit ist.

12. Verfahren zur Verbesserung der Qualität von Eizellen oder Blastozysten, umfassend das Inkontaktbringen der Eizelle oder Blastozyste mit einer wirksamen Menge eines Salzes nach einem der Ansprüche 1 bis 9 oder der Zusammensetzung nach Anspruch 10 vor der Implantation in ein Subjekt, das einer Behandlung von altersbedingter Unfruchtbarkeit bedarf, wobei die Eizelle oder Blastozyste gegebenenfalls in einem IVF-Medium kultiviert wird, das das Salz enthält.

13. Verfahren zur Verbesserung der Eizellen- oder Blastozystenreifung, umfassend das Inkontaktbringen der Eizelle oder Blastozyste mit einer wirksamen Menge eines Salzes nach einem der Ansprüche 1 bis 9 oder der Zusammensetzung nach Anspruch 10 vor der Implantation in ein Subjekt, das einer Behandlung der altersbedingten Unfruchtbarkeit bedarf,
wobei die Eizelle oder Blastozyste gegebenenfalls in einem IVF-Medium kultiviert wird, das das Salz enthält.

14. Zellkulturmedium für die *In-vitro*-Fertilisation, umfassend:
eine Verbindung, ausgewählt aus einem der Ansprüche 1 bis 9; und
einem Kultivierungsmittel.

15. Zellkulturmedium für die *In-vitro*-Fertilisation nach Anspruch 14, wobei das Kulturmedium ein anorganisches Salz, ein Energiesubstrat, eine Aminosäure, ein Chelatbildner, ein pH-Indikator, ein Antibiotikum, ein Serum, ein Vitamin, ein Wachstumsfaktor oder eine beliebige Kombination davon ist; wobei gegebenenfalls:
(a) das anorganische Salz Calciumchlorid, Magnesiumchlorid, Magnesiumsulfat, Kaliumchlorid, Natriumbicarbonat, Natriumchlorid, Mononatriumphosphat, Dinatriumphosphat oder eine beliebige Kombination davon ist,
(b) das Energiesubstrat Glukose, Pyruvat, Laktat, Pyruvat oder eine beliebige Kombination davon ist,
(c) die Aminosäure eine essentielle Aminosäure, gegebenenfalls Arginin, Cystein, Glutamin, Glycin, Histidin, Isoleucin, Leucin, Lysin, Methionin, Phenylalanin, Prolin, Threonin, Tryptophan, Tyrosin, Valin oder eine beliebige Kombination davon, ist,
(d) die Aminosäure eine nicht essentielle Aminosäure, gegebenenfalls Alanin, Asparagin, Aspartat, Glutamat, Prolin, Serin oder eine beliebige Kombination davon, ist
(e) der Chelatbildner Clathrochelat, Acetylaceton, Aminopolycarbonsäure, ATMP, BAPTA, BDTH2, Zitronensäure, Kryptand, Deferasirox, 2,3-Dihydrobenzoesäure, 2,3-Dimercapto-1-propansulfonsäure, Dimercaptobernsteinsäure, DOTA, DTPMP, EDDHA, EDDS, EDTMP, Etidronsäure, Fura-2, Gluconsäure, Homozitronensäure, Iminodiessigsäure, Indo-1, Nitril-Triessigsäure, Pentetsäure (DTPA), Phosphonat, Phytochelati, Polyasparaginsäure, Natriumpolyaspartat, Trinatriumcitrat, Transferrin, EDTA, EGTA oder eine beliebige Kombination davon ist,
(f) der pH-Indikator Phenolrot, Bromthymolblau, Alizarinrot, 9-Aminoacridin oder eine beliebige Kombination davon ist,
(g) das Antibiotikum Actinomycin D, Ampicillin, Carbenicillin, Cefotaxim, Fosmidomycin, Gentamicin, Kanamycin, Neomycin, Penicillin, Polymyxin B, Streptomycin oder eine beliebige Kombination davon ist, oder
(h) das Serum Humanalbumin, Rinderserumalbumin, fötales Rinderserum, synthetisches Serum oder eine beliebige Kombination davon ist.

## Revendications

1. Sel de formule (I) : 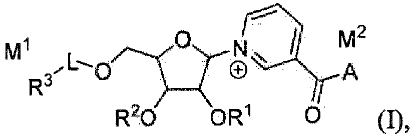
ou énantiomère, stéréoisomère ou tautomère de celui-ci,
dans lequel
A est NR^{a}R^{b} ou O⁻ ;
L est une liaison ou 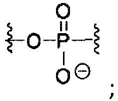
R¹ et R² sont indépendamment H, alkyle en C₁-C₆, haloalkyle en C₁-C₆, - C(O)haloalkyle en C₁-C₆, (alkylène en C₀-C₃)C(O)alkyle en C₁-C₆, -C(O)OR^{a}, - C(O)NR^{a}R^{b},-[CH₂-CH₂-O]ₖ-R^{a}, -C(O)[CH₂-CH₂-O]ₖ-R^{a}, -[CH₂-CH₂-CH₂-O]ₖ-R^{a} ou - C(O)[CH₂-CH₂-CH₂-O]ₖ-R^{a},
ou R¹ et R², avec l'atome auquel ils sont liés, forment un cycle hétérocyclique à 5 chaînons facultativement substitué par un ou plusieurs substituants choisis parmi alkyle en C₁-C₆, alcényle en C₂-C₆, alcynyle en C₂-C₆, (alkylène en C₀-C₃)cycloalkyle en C₃-C₈, (alkylène en C₀-C₃)hétérocycloalkyle, (alkylène en C₀-C₃)aryle en C₆-C₁₄ ou (alkylène en C₀-C₃)hétéroaryle ;
R³ est une charge négative, H ou alkyle en C1-C₆ ;
R^{a} et R^{b} sont indépendamment, à chaque occurrence, H ou alkyle en C₁-C₆, dans lequel l'alkyle est facultativement substitué par un ou plusieurs substituants choisis parmi (alkylène en C₀-C₃)cycloalkyle en C₃-C₈, (alkylène en C₀-C₃)hétérocycloalkyle, (alkylène en C₀-C₃)aryle en C₆-C₁₄ ou (alkylène en C₀-C₃)hétéroaryle ; et
k est un nombre entier de 1 à 8,
M¹ et M² sont indépendamment absents ou un cation quaternaire de formule (II) : 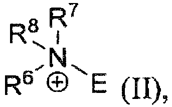 dans lequel
E est un alkyle en C₁-C₆ facultativement substitué par un ou plusieurs substituants choisis parmi R⁴, R⁵, G-OR⁹ ou (alkylène en C₀-C₃)OR¹¹ ;
R⁴ et R⁵ sont indépendamment, à chaque occurrence, H ou alkyle en C₁-C₆, alcényle en C₂-C₆, alcynyle en C₂-C₆, (alkylène en C₀-C₃)cycloalkyle en C₃-C₈, (alkylène en C₀-C₃)hétérocycloalkyle, (alkylène en C₀-C₃)aryle en C₆-C₁₄ ou (alkylène en C₀-C₃)hétéroaryle, dans lequel l'alkyle, l'alcényle, l'alcynyle, l'alkylène, le cycloalkyle, l'hétérocycloalkyle, l'aryle ou l'hétéroaryle est facultativement substitué par un ou plusieurs substituants choisis parmi cyano, halo, SeH, (alkylène en C₀-C₃)NR^{c}R^{d}, (alkylène en C₀-C₃)OR^{c}, (alkylène en C₀-C₃)OC(O)R^{c}, (alkylène en C₀-C₃)C(O)OR^{c}, (alkylène en C₀-C₃)SR^{c}, (alkylène en C₀-C₃)C(O)SR^{c}, (alkylène en C₀-C₃)SC(O)R^{c}, (alkylène en C₀-C₃)C(O)NR^{c}R^{d}, (alkylène en C₀-C₃)NC(O)NR^{c}R^{d}, (alkylène en C₀-C₃)C(NR^{c})NR^{c}R^{d}, (alkylène en C₀-C₃)NR^{c}C(NR^{c})NR^{c}R^{d}, (alkylène en C₀-C₃)P(O)OₙR^{c}R^{d}, (alkylène en C₀-C₃)S(O)ₘNR^{c}R^{d}, (alkylène en C₀-C₃)S(O)ₘOR^{c} ou (alkylène en C₀-C₃)BOₚR^{c}R^{d} ;
R⁶, R⁷ et R⁸ sont indépendamment H ou alkyle en C₁-C ₆, dans lequel l'alkyle est facultativement substitué par un ou plusieurs substituants choisis parmi cyano, halo, (alkylène en C₀-C₃)NR^{c}R^{d} ou (alkylène en C₀-C₃)OR^{c},
ou R⁴ et R⁶, avec les atomes auxquels ils sont liés, forment un cycle à 5 à 6 chaînons facultativement substitué par un ou plusieurs substituants choisis parmi cyano, halo, (alkylène en C₀-C₃)NR^{c}R^{d} ou (alkylène en C₀-C₃)OR^{c},
ou R⁷ et R⁸, avec les atomes auxquels ils sont liés, forment un cycle à 5 à 6 chaînons facultativement substitué par un ou plusieurs substituants choisis parmi cyano, halo, (alkylène en C₀-C₃)NR^{c}R^{d} ou (alkylène en C₀-C₃)OR^{c} ;
R^{c} et R^{d} sont indépendamment, à chaque occurrence, H ou alkyle en C₁-C₆, dans lequel l'alkyle est facultativement substitué par un ou plusieurs substituants choisis parmi (alkylène en C₀-C₃)cycloalkyle en C₃-C₈, (alkylène en C₀-C₃)hétérocycloalkyle, (alkylène en C₀-C₃)aryle en C₆-C₁₄ ou (alkylène en C₀-C₃)hétéroaryle ;
R⁹ est une charge négative, H ou alkyle en C₁-C₆ ;
R¹¹ est H, alkyle en C₁-C₆ ou C(O)alkyle en C₁-C ₆, dans lequel l'alkyle en C₁-C₆ ou C(O)alkyle en C₁-C₆ est facultativement substitué par un ou plusieurs substituants choisis parmi (alkylène en C₀-C₃)NR^{c}R^{d}, (alkylène en C₀-C₃)OR^{c}, (alkylène en C₀-C₃)cycloalkyle en C₃-C₈, (alkylène en C₀-C₃)hétérocycloalkyle, (alkylène en C₀-C₃)aryle en C₆-C₁₄ ou (alkylène en C₀-C₃)hétéroaryle ;
G est CH₂ ou C(O) ; et
m, n et p sont indépendamment, à chaque occurrence, 0, 1 ou 2 ;
à condition que
a) au moins un parmi M¹ ou M² soit un cation quaternaire
b) lorsque L est une liaison, M¹ soit absent, et
c) lorsque A est NR^{a}R^{b}, M² soit absent.

2. Sel selon la revendication 1, dans lequel A est O⁻.

3. Sel selon la revendication 1, dans lequel A est NR^{a}R^{b}, facultativement dans lequel R^{a} est H ou méthyle.

4. Sel selon l'une quelconque des revendications 1 à 3, dans lequel M¹ et M² sont indépendamment absents ou un cation ammonium quaternaire de formule (IIa) : 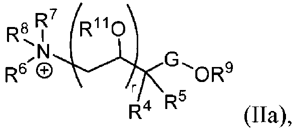 dans lequel r est 0 ou 1.

5. Sel selon l'une quelconque des revendications 1 à 4, dans lequel R⁵ est H et/ou R⁶ est H.

6. Sel selon l'une quelconque des revendications 1 à 5, dans lequel R⁴ est H, ou
dans lequel R⁴ est alkyle en C₁-C₄, (alkylène en C₀-C₃)aryle en C₆-C₁₄ ou (alkylène en C₀-C₃)hétéroaryle ou alkyle en C₁-C₄ substitué par un ou plusieurs (alkylène en C₀-C₃)SR^{c}.

7. Sel selon l'une quelconque des revendications 1 à 6, dans lequel M¹ est 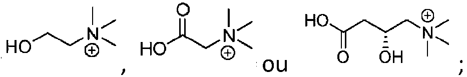 ou dans lequel M¹et M² sont

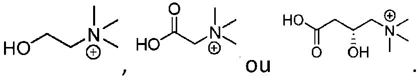

8. Sel selon l'une quelconque des revendications 1 à 7, dans lequel R¹ est H, C(O)alkyle en C₁-C₃ ou alkyle en C₁-C₃ ; et/ou dans lequel R² est H, C(O)alkyle en C₁-C₃ ou alkyle en C₁-C₃ ;
et/ou dans lequel R³ est H ou méthyle.

9. Sel selon la revendication 1, ayant la structure 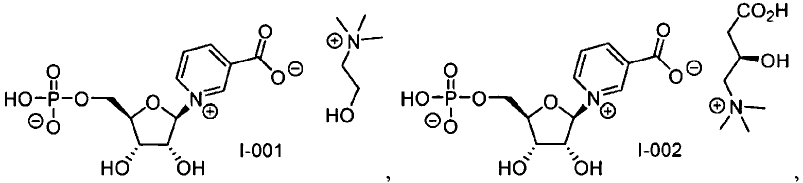 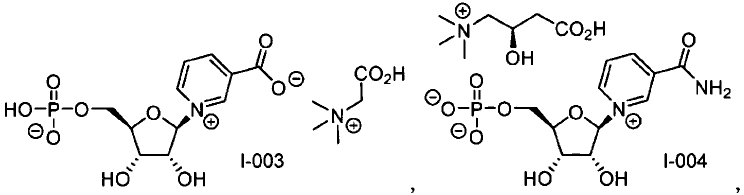 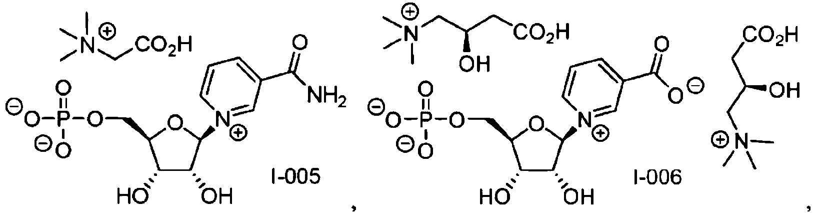 ou

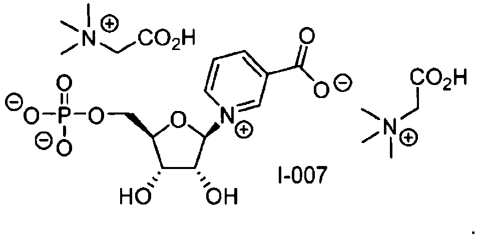

10. Composition pharmaceutique comprenant un sel selon l'une quelconque des revendications 1 à 9 et un vecteur pharmaceutiquement acceptable.

11. Sel selon l'une quelconque des revendications 1 à 9 ou composition selon la revendication 10, pour une utilisation dans un procédé de traitement ou de prévention de l'infertilité, le procédé comprenant l'administration à un sujet en ayant besoin d'une quantité efficace du sel ou de la composition,
facultativement dans lequel l'infertilité est une infertilité liée à l'âge.

12. Procédé d'amélioration de la qualité d'ovocyte ou de blastocyste comprenant la mise en contact de l'ovocyte ou du blastocyste avec une quantité efficace d'un sel selon l'une quelconque des revendications 1 à 9 ou la composition selon la revendication 10 avant implantation chez un sujet ayant besoin d'un traitement de l'infertilité liée à l'âge, dans lequel l'ovocyte ou le blastocyste est facultativement mis en culture dans un milieu de FIV contenant le sel.

13. Procédé d'amélioration de la maturation d'ovocyte ou de blastocyte comprenant la mise en contact de l'ovocyte ou du blastocyste avec une quantité efficace d'un sel selon l'une quelconque des revendications 1 à 9 ou la composition selon la revendication 10 avant implantation chez un sujet ayant besoin d'un traitement de l'infertilité liée à l'âge, dans lequel l'ovocyte ou le blastocyste est facultativement mis en culture dans un milieu de FIV contenant le sel.

14. Milieu de culture cellulaire pour fécondation in vitro comprenant :
un composé choisi parmi l'une quelconque des revendications 1 à 9 ; et
un agent de culture.

15. Milieu de culture cellulaire pour fécondation in vitro selon la revendication 14, dans lequel l'agent de culture est un sel inorganique, un substrat énergétique, un acide aminé, un chélateur, un indicateur de pH, un antibiotique, un sérum, une vitamine, un facteur de croissance ou toute combinaison de ceux-ci ; facultativement dans lequel :
(a) le sel inorganique est chlorure de calcium, chlorure de magnésium, sulfate de magnésium, chlorure de potassium, bicarbonate de sodium, chlorure de sodium, phosphate monosodique, phosphate disodique ou toute combinaison de ceux-ci,
(b) le substrat énergétique est glucose, pyruvate, lactate, pyruvate ou toute combinaison de ceux-ci,
(c) l'acide aminé est un acide aminé essentiel, facultativement arginine, cystéine, glutamine, glycine, histidine, isoleucine, leucine, lysine, méthionine, phénylalanine, proline, thréonine, tryptophane, tyrosine, valine ou toute combinaison de ceux-ci,
(d) l'acide aminé est un acide aminé non essentiel, éventuellement alanine, asparagine, aspartate, glutamate, proline, sérine ou toute combinaison de ceux-ci,
(e) le chélateur est clathrochélate, acétylacétone, acide aminopolycarboxylique, ATMP, BAPTA, BDTH2, acide citrique, cryptand, déférasirox, acide 2,3-dihydrobenzoïque, acide 2,3-dimercapto-1-propane sulfonique, acide dimercapto succinique, DOTA, DTPMP, EDDHA, EDDS, EDTMP, acide étidronique, fura-2, acide gluconique, acide homocitrique, acide iminodiacétique, Indo-1, acide nitrile triacétique, acide pentétique (DTPA), phosphonate, phytochélatine, acide polyaspartique, polyaspartate de sodium, citrate trisodique, transferrine, EDTA, EGTA ou toute combinaison de ceux-ci,
(f) l'indicateur de pH est rouge de phénol, bleu de bromothymol, rouge d'alizarine, 9-aminoacridine ou toute combinaison de ceux-ci,
(g) l'antibiotique est actinomycine D, ampicilline, carbénicilline, céfotaxime, fosmidomycine, gentamicine, kanamycine, néomycine, pénicilline, polymyxine B, streptomycine ou toute combinaison de ceux-ci, ou
(h) le sérum est albumine sérique humaine, albumine sérique bovine, sérum fœtal bovin, sérum synthétique ou toute combinaison de ceux-ci.
